(19) Europäisches Patentamt / European Patent Office / Office européen des brevets

(11) **EP 2 172 162 B1**

(12) **EUROPEAN PATENT SPECIFICATION**

(45) Date of publication and mention
of the grant of the patent:
**06.03.2019 Bulletin 2019/10**

(51) Int Cl.:
***A61B 18/18*** *(2006.01)*

(21) Application number: **09172188.6**

(22) Date of filing: **05.10.2009**

(54) **Combined frequency microwave ablation system und devices**

Kombiniertes Frequenzmikrowellenablationssystem und Vorrichtungen

Système d'ablation de micro-ondes de fréquence combinée et dispositifs

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **03.10.2008 US 244850**

(43) Date of publication of application:
**07.04.2010 Bulletin 2010/14**

(73) Proprietor: **Covidien LP
Mansfield, MA 02048 (US)**

(72) Inventor: **Paulus, Joseph A.
Louisville, CO 80027 (US)**

(74) Representative: **Soames, Candida Jane et al
Maschio & Soames IP Limited
30 Carlton Crescent
Southampton SO15 2EW (GB)**

(56) References cited:
**WO-A2-03/039385       WO-A2-2009/040523
CN-A- 1 103 807       DE-A1- 10 310 765
JP-A- 2001 231 870       US-A- 4 448 198
US-A1- 2005 205 566**

## Description

## BACKGROUND

## Technical Field

The present invention relates generally to medical / surgical ablation systems for delivering microwave energy to tissue. More particularly, the present disclosure relates to the spectral frequency content of the energy delivered to tissue to achieve deep penetration of energy.

## Background of Related Art

In the treatment of diseases such as cancer, certain types of cancer cells have been found to denature at elevated temperatures (which are slightly lower than temperatures normally injurious to healthy cells). These types of treatments, known generally as hyperthermia therapy, typically utilize electromagnetic radiation to heat diseased cells to temperatures above 41° C while maintaining adjacent healthy cells at lower temperatures to insure that irreversible cell destruction does not occur. Other procedures utilizing electromagnetic radiation to heat tissue also include ablation and coagulation of the tissue. Such microwave ablation procedures, e.g., such as those performed for menorrhagia, are typically performed to ablate and coagulate the targeted tissue to denature or kill the tissue. Many procedures and types of devices utilizing electromagnetic radiation therapy are known in the art. Such microwave therapy is typically used in the treatment of tissue and organs, such as the prostate, heart, and liver.

One less invasive procedure generally involves the treatment of tissue (e.g., a tumor) underlying the skin via the use of microwave energy. While microwave energy is able to penetrate the skin to reach the underlying tissue, the depth of penetration is typically dependant on several factors such as the physical properties of the tissue, the type of ablation instrument used for ablation, the current density pattern generated by the ablation instrument and the rate of energy delivery to tissue, and the spectral content of the energy.

The first factor, the physical properties of the tissue, is determined by the target tissue to be ablated, (i.e., the cancerous tissue) and the healthy tissue surrounding the target tissue. Obviously, a clinician cannot control the size or shape of the target tissue, or the location of the target tissue in the patient's body, but a clinician can select the type and number of ablation instruments, adjust the amount of delivered power, adjust the rate of energy delivery and vary the spectral content of the microwave energy.

In a conventional system, the spectral content of the energy is fixed to a particular frequency, such as, for example, 915 MHz, 2450 MHz and 10 GHz. The spectral content of the microwave signal determines the current density along the antenna and the amount of microwave energy delivered to the surrounding medium typically determines the depth of energy penetration and the shape of the resulting ablation region. For example, it is well known that a device delivering energy at 10 GHz only penetrates tissue a few millimeters while a device delivering energy at 915 MHz may penetrate tissue several centimeters.

Delivery of microwave energy at a single microwave frequency provides specific advantages and disadvantages. The present disclosure overcomes disadvantages of delivering energy at a specific frequency by disclosing an electrosurgical system, device and methods to simultaneously delivery microwave energy at a plurality of microwave frequencies which may allow better control over energy delivery and deposition around the instrument, as well as potentially sensing or determining the ablation shape or completeness by using antenna matching at different spectral combinations. US2005205566 A1, US4448198 A1 disclose systems similar to the present invention.

## SUMMARY

The present invention is defined by independent claim 1. Preferred embodiments are disclosed in the dependent claims. A system for delivering energy is disclosed. The system includes a housing having an antenna attached to the distal end configured to receive a microwave signal and radiate energy at two or more wavelengths and a microwave generator operably connecting to the antenna that provides the microwave signal to the antenna. The microwave generator generates a combined microwave signal containing microwave energy having at least a first and a second wavelength. The at least a first and second wavelengths are both capable of creating resonance in the antenna.

The system includes a first microwave signal generator that generates a first microwave signal at the first wavelength, a second microwave signal generator that generates a second microwave signal at the second wavelength and a signal mixer that combines the first and second microwave signals to generate the combined microwave signal. The first wavelength is related to a first frequency and the second wavelength is related to a harmonic of the first frequency. The harmonic may be a third or a fifth harmonic. The first frequency may be about 915 MHz. The system includes a phase shifter configured to shift the phase of one of the first and second microwave signals relative to each other. The phase shifter may shift the phase between the first and second microwave signals between about 0° and 360°.

In one embodiment, the system also includes an amplifier that amplifies at least one of the first and second microwave signals to a desired intermixing ratio. The mixing ratio may be between about 1:99 and 99: An amplifier is provided to amplify the combined microwave signal. In yet another embodiment, the system may further include a processor configured to control a parameter of the first microwave signal, the second microwave signal or the combined signal.

## BRIEF DESCRIPTION OF THE DRAWINGS

Various embodiments of the present disclosure are described herein with reference to the drawings wherein:

FIG. 1 is a perspective view of an electrosurgical system according to an embodiment of the present disclosure;

FIG. 2 is a transverse cross-sectional view of the distal end of the microwave energy delivery device of FIG. 1;

FIG. 3 is a graphical illustration of the half-wave dipole antenna portion of FIG. 2, with an illustration of the corresponding current density created along the antenna when driven by microwave signals with wavelengths of $\lambda$;

FIG. 4 is a graphical illustration of the half-wave dipole antenna portion of FIG. 3, with an illustration of the corresponding current density created along the antenna when driven by microwave signals with wavelengths of $\lambda$, $\lambda_{3rd}$, and $\lambda_{5th}$;

FIG. 5 is a block diagram of the microwave energy generation circuit from FIG. 1 for generating and combining microwave signals with two or more wavelengths, according to the present invention;

FIG. 6A is a graphical illustration of the waveforms $\lambda$ and $\lambda_{3th}$, with time represented as the 'phase angle' of the $\lambda$ waveform with the magnitude of the two waveforms normalized;

FIG. 6B is a time series of graphical illustrations of current density patterns at instantaneous points in time generated by an antenna driven by waveforms $\lambda$ and $\lambda_{3th}$ from FIG. 6A and the resultant current density waveform;

FIG. 7 is a graphical illustration of the half-wave dipole antenna portion of FIG. 2, with an illustration of the corresponding current density created along the antenna when driven by microwave signals with wavelengths of $\lambda$, $\lambda_{3rd}$ and the resultant current density waveform;

FIG. 8A is a graphical illustration of the waveforms $\lambda$ and $\lambda_{3th}$, with time represented as the 'phase angle' of the $\lambda$ waveform, the two waveforms phase shifted by 30° relative to the $\lambda$ waveform with the magnitude of the two waveforms normalized;

FIG. 8B is a time series of graphical illustrations of current density patterns at instantaneous points in time generated by an antenna driven by waveforms $\lambda$ and $\lambda_{3th}$ from FIG. 8A and the resultant current density waveform;

FIG. 9A is a graphical illustration of the waveforms $\lambda$ and $\lambda_{3th}$, with time represented as the 'phase angle' of the $\lambda$ waveform, the two waveforms phase shifted by 60° relative to the $\lambda$ waveform with the magnitude of the two waveforms normalized;

FIG. 9B is a time series of graphical illustrations of current density patterns at instantaneous points in time generated by an antenna driven by waveforms $\lambda$ and $\lambda_{3th}$ from FIG. 9A and the resultant current density waveform;

FIG. 10 is a graphical illustration of the half-wave dipole antenna portion of FIG. 2, with an illustration of the corresponding current density created along the antenna when driven by microwave signals with wavelengths of $\lambda$, $\lambda_{5th}$ and the resultant current density waveform;

FIG. 11 is a graphical illustration of the half-wave dipole antenna portion of FIG. 2, with an illustration of the corresponding current density created along the antenna when driven by microwave signals with wavelengths of $\lambda$, $\lambda_{3rd}$ phase shifted by 180° and the resultant current density waveform;

FIG. 12 is a graphical illustration of the half-wave dipole antenna portion of FIG. 2, with an illustration of the corresponding current density created along the antenna when driven by microwave signals with wavelengths of $\lambda_{3rd}$, $\lambda_{5th}$ phase shifted by 180° and the resultant current density waveform;

FIG. 13 is a graphical illustration of the half-wave dipole antenna portion of FIG. 2, with an illustration of the corresponding current density created along the antenna when driven by microwave signals with wavelengths of $\lambda_{3rd}$, $\lambda_{5th}$ and the resultant current density waveform wherein the waveform $\lambda_{3rd}$ and $\lambda_{5th}$ are a 3:1. intensity level;

FIG. 14 is a graphical illustration of the half-wave dipole antenna portion of FIG. 2, with an illustration of the corresponding current density created along the antenna when driven by microwave signals with wavelengths of $\lambda_{3rd}$, $\lambda_{5th}$ phase shifted by 180° and the resultant current density waveform; and

FIG. 15 is a graphical illustration of the half-wave dipole antenna portion of FIG. 2, with an illustration of the corresponding current density created along the antenna when driven by microwave signals with

wavelengths of $\lambda_{3rd}$, $\lambda_{5th}$ phase shifted by 180° and resultant current density waveform wherein the waveform $\lambda_{3rd}$ and $\lambda_{5th}$ are a 3:1 intensity level.

## DETAILED DESCRIPTION

Embodiments of the presently disclosed microwave antenna assembly are described in detail with reference to the drawing figures wherein like reference numerals identify similar or identical elements. As used herein and as is traditional, the term "distal" refers to the portion which is furthest from the user and the term "proximal" refers to the portion that is closest to the user. In addition, terms such as "above", "below", "forward", "rearward", etc. refer to the orientation of the figures or the direction of components and are simply used for convenience of description.

During treatment of diseased areas of tissue in a patient, the insertion and placement of an microwave energy delivery apparatus, such as a microwave antenna assembly, relative to the diseased area of tissue is preferable for successful treatment. Generally, the microwave antenna assemblies described herein allow for direct insertion into tissue and include a half-wave dipole antenna at the distal end. An microwave assembly for percutaneous insertion into tissue is described in U.S. Patent No. 6,878,147 to Prakash, issued on April 12, 2005.

One critical aspect of placement of a microwave energy delivery apparatus is determining the size and shape of the ablation area produced by the device and insuring that the target tissue is contained within this ablation area.

*Microwave Energy Ablation System*

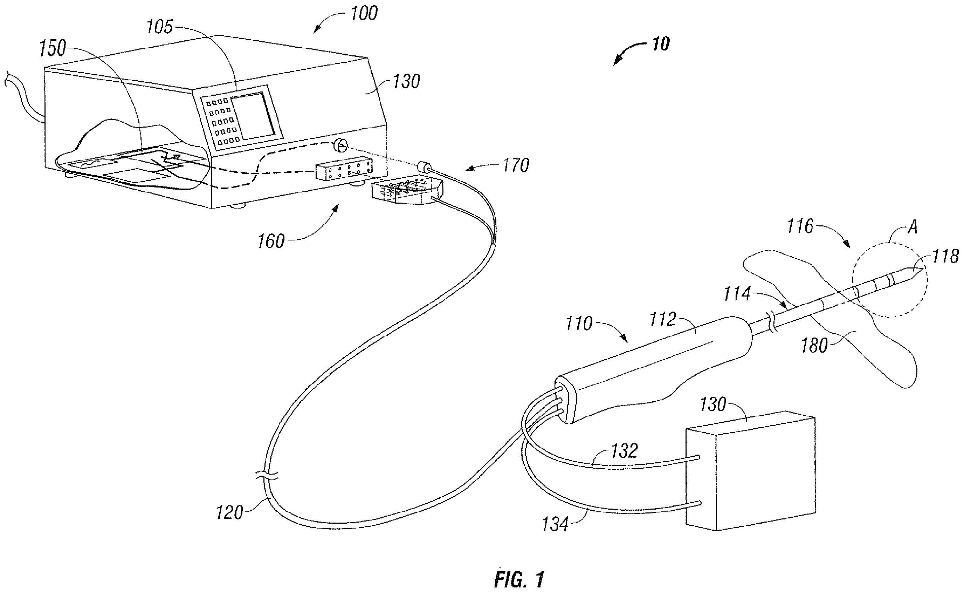

Referring now to FIG. 1, a combined frequency microwave ablation system (hereinafter "microwave ablation system"), according to an embodiment of the present disclosure, is shown as system 10. Microwave ablation system 10 includes a combined frequency microwave generator 100 (hereinafter "microwave generator") connected to a microwave energy delivery device 110 via a transmission line 120 and, in some embodiments, a cooling fluid supply 130. Microwave generator 100 includes a housing 130 that houses a microwave energy generation circuit 150 and a delivery device port 160 that connects to the device connector 170 of the transmission line 120.

Microwave energy delivery device 110 includes a handle 112 and an elongate shaft 114 including an antenna 116 on the distal end. Distal portion of antenna 116 may form a sharpened tip 118 for percutaneous insertion into patient tissue 180. If present, a cooling fluid supply 130 supplies cooling fluid to microwave energy delivery device 110 via supply and return tubes 132, 134, respectively, connected to the proximal end of handle 112.

Microwave energy delivery device 110 may be designed and intended for use with a conventional system that supplies microwave energy at a single microwave frequency or microwave energy delivery device may be specifically designed and intended for use with a combined frequency microwave generator 100. While the present disclosure describes a combined frequency microwave ablation system 10 and methods of use with a percutaneous type microwave energy delivery device, the systems and methods disclosed herewithin may be used with any suitable microwave energy delivery device 110 capable of delivering microwave energy, such as, for example, a catheter-type device, an endoscopic device and a surface delivery device (not shown).

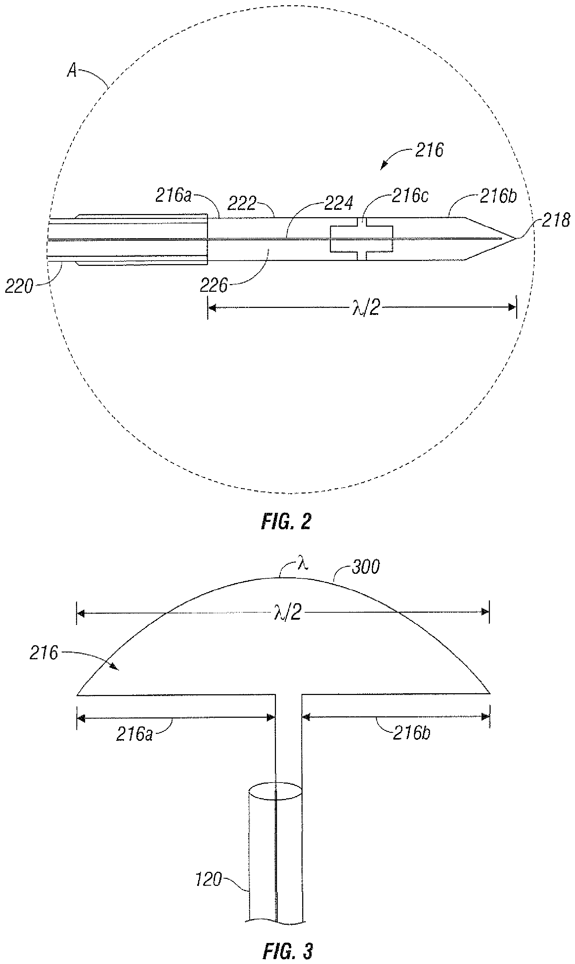

FIG. 2 is a transverse cross-sectional view of the distal end of a microwave energy delivery device 110 similar to the device of FIG. 1. Antenna 216 is a conventional half-wave dipole microwave antenna. Antenna 216 includes a proximal radiating portion 216a connected to a feedline 220 at the proximal end thereof, and a distal radiating portion 216b. Sharpened tip 218 may be part of the distal radiating portion 216b or sharpened tip 218 may connect to distal radiating portion 216b and configured to not radiate energy. Antenna 216 comprises proximal radiating portion 216a and distal radiating portion 216b. Proximal radiating portion 216a may typically have an outer conductor 222 and an inner conductor 224, each of which extends along a longitudinal axis. Between the outer and inner conductors 222, 224 is typically a dielectric material 226 that is disposed longitudinally between the conductors 222, 224 to provide electrical isolation therebetween. Dielectric material 226 may constitute any number of appropriate materials, including air. Proximal and distal radiating portions 216a, 216b align at junction 216c, typically formed of a dielectric material. Junction 216c may be supported by inner conductor 224 that runs through junction 216c and at least partially extends into distal radiating portion 216b.

In use, a conventional system generates a microwave energy signal having a wavelength of $\lambda$ and transmits the signal through a transmission line 220 to antenna 216. Antenna 216 transmits energy from the proximal and distal radiating portions 216a, 216b. The radiating portions produce an electric field (hereinafter "E-field") in the surrounding medium which agitates and/or rotates H20 molecules (and other polar molecules) to produce heat.

In general, an E-field, generated by the time-varying magnetic field, exerts a force on other electrically charged objects. While "E-fields" and "magnetic fields" are not the same, they cannot be completely separable. Therefore, for the sake of clarity the term "electromagnetic field" will be used to describe an E-field, a magnetic field or the combined forces generated by either an E-field or magnetic field. The electromagnetic field may produce localized movement of H20 molecules, i.e., vibrational and/or rotational movement, or the electromagnetic field may induce current flow over very short distances.

This agitation, friction and/or induced current between molecules produces heat in the surrounding medium. The strength of the E-field at a given point is defined as the force that would be exerted on a charge at any given that point and the direction of the E-field is given by the direction of that force.

The strength of the electromagnetic field may be represented by the current density (i.e., the measure of the density of a conserved charge) in the surrounding tissue. While current density is typically related to electric current, in the present disclosure current density generally represents the magnitude and relative strength of the electromagnetic field generated when a microwave signal is applied to the antenna.

The physical length of the antenna 216 for efficient radiation of microwave energy may depend on several factors. One factor is the effective wavelength, $\lambda_{eff}$, which is dependent upon the dielectric properties of the surrounding medium. Antenna 216 through which microwave energy is transmitted at a wavelength, $\lambda$, may have differing effective wavelengths, $\lambda_{eff}$, depending upon the surrounding medium, e.g., liver tissue, as opposed to, e.g., breast tissue. Also affecting the effective wavelength, $\lambda_{eff}$, are coatings which may be disposed over antenna 216.

For simplicity, in the present disclosure a wavelength of the signal generated by the microwave generator and supplied to the antenna 216 is generically referenced as $\lambda$, wherein the physical length of the antenna 216 is approximately about one-half the wavelength of the microwave signal, or $\lambda/2$, and is the "effective' $\lambda$ of the antenna in the respective media.

FIG. 3 is the half-wave di-pole antenna 216 of FIG. 2, with a graphical illustration of the corresponding instantaneous current density 300 along the antenna 216 when supplied with a microwave signal at a wavelength of $\lambda$. Antenna 216 includes proximal and distal radiating portions 216a, 216b with a sinusoidal current distribution across the antenna 216. The maximum current is at the center of the di-pole with zero current density at each end. The current density 300 is a representation of the instantaneous relative energy penetration in the surrounding medium related to the electromagnetic field patterns / waves radiated into surrounding tissue.

While the antenna 216 illustrated in FIG. 3 is a balanced di-pole antenna wherein the proximal and distal radiating portions 216a, 216b are substantially equal, the embodiments described herein may be implemented with an unbalanced di-pole antenna (wherein the proximal and distal radiating portions 216a, 216b are not equal.)

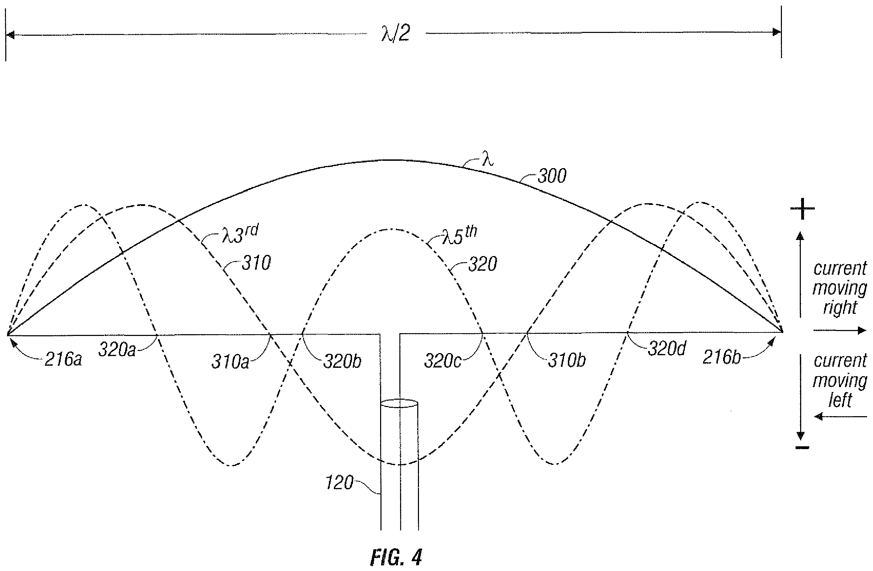

Antenna 216 may also resonate and deliver energy to the surrounding medium when driven with signals containing additional wavelengths, wherein the wavelengths are odd multiple harmonics of $\lambda$. As illustrated in FIG. 4, a half-wave di-pole antenna with a physical length approximately equal to $\lambda/2$ will also resonate with wavelengths of $\lambda$, $\lambda_{3rd}$ 310 and $\lambda_{5th}$ 320, wherein $\lambda_{3rd}$ 310 and $\lambda_{5th}$ 320 are third and fifth harmonics of $\lambda$, respectively. The physical length of the antenna is equal to $\lambda/2$ for a wavelength of $\lambda$ 300, $3\lambda_{3rd}/2$ for a wavelength of $\lambda_{3rd}$ 310 and $5\lambda_{5th}/2$ for a wavelength of $\lambda_{5th}$ 320. The current densities for $\lambda$ 300, $\lambda_{3rd}$ 310 and $\lambda_{5th}$ 320 are sinusoidal in shape with $\lambda$ 300 having a single sinusoidal node, $\lambda_{3rd}$ having three sinusoidal nodes and $\lambda_{5th}$ 320 having five sinusoidal nodes.

When supplying energy to antenna 216 at a single wavelength, such as, for example, $\lambda$, $\lambda_{3rd}$ or, $\lambda_{5th}$, heating of the surrounding medium is dependant on the current density and the current density is similar in shape to the absolute value of the waveform 300, 310, 320.

As illustrated in FIG. 3, providing a microwave signal, with a wavelength of $\lambda$ and a frequency of 915 MHz, to an antenna 216 with a physical length of approximately $\lambda/2$ will generate current 300 in a sinusoidal distribution. The current 300 relates to the electromagnetic field and results in heating of the surrounding medium. The size of the resulting ablation area is related to the magnitude of the current 300 thus forming a circular ablation region. Ablation region may also be described as a donut or torus shaped region.

As illustrated in FIG. 4, an antenna 216 with a length of $\lambda/2$ will also resonate and delivery energy into the surrounding medium when supplied with the third harmonic $\lambda_{3rd}$ 310 of $\lambda$ and the fifth harmonics $\lambda_{5th}$ 320 of $\lambda$. For example, an antenna that resonates at about 915 MHz, or $\lambda$ 300, will also resonate at about 2.525 GHz, or $\lambda_{3rd}$, and at 4.209 GHz, or $\lambda_{5th}$. For illustrative purposes, the sine waves and other waveforms described herein relate to current density in a direction at a specific moment in time, wherein a portion of the waveform above the dipole provides the magnitude of the current moving to the right and a portion of the waveform below the dipole provides the magnitude of the current moving to the left.

At wavelengths of $\lambda_{3rd}$ and $\lambda_{5th}$ the magnitude of current 310, 320, respectively, along the antenna 216 is different than the magnitude of the current at $\lambda$ 300, thereby resulting in a different penetration pattern of energy into the surrounding medium. The actual shape and size of each ablation region may also be dependant on elements, such as, for example, the physical properties of the surrounding medium and the amount of energy delivered at each wavelength or harmonic.

Lower microwave frequencies typically provide deeper penetration of energy into the surrounding medium with less immediate or less concentrated damage to the medium as compared to higher microwave frequency microwaves. Alternatively, energy delivery at higher frequencies may be less susceptible to changes in tissue properties and antenna resonant shifting and generally deliver more localized energy. In one embodiment of the present disclosure, the microwave generator 100 of FIG. 1 supplies microwave energy to the microwave energy delivery device 110 at two or more frequencies wherein the two or more frequencies are resonant frequencies of the antenna 116. For example, microwave generator may delivery energy with a first wavelength, such as, for example, $\lambda$ and a second wavelength, such as, for example, one or both $\lambda_{3rd}$ and $\lambda_{5th}$. The frequencies may be selected to provide favorable resonance matching at one or more of the selected frequencies.

In an arrangement not part of the invention, an ablation procedure includes a microwave generator 100 for supplying energy at a first frequency such as, for example, 915 MHz, with a corresponding wavelength of $\lambda$, for a first period of time, switching to a second frequency such as, for example 2.450 GHz, with a corresponding wavelength of $\lambda_{3rd}$, for a second period of time and return to a first frequency for a third period of time. Microwave generator 100 may determine the best relationship between the two frequencies, frequency combinations and/or the duration of each delivery period (i.e., s-parameter relationship such as, for example, the S11, or input port voltage reflection coefficient, for each frequency).

In an arrangement not part of the invention, an ablation procedure includes a microwave generator 100 supplying energy at a first frequency for a first period of time, a second frequency for a second period of time and a third frequency for a third period of time.

A measured parameter may be used to determine one or more of the delivered frequencies.

*Combined Frequency Microwave Ablation System*

The present invention relates to systems, devices and methods for combining resonant frequencies simultaneously during ablation in order to achieve deeper penetration or variably targeting of microwave energy.

With continued reference to FIG. 4, it can be appreciated that at a wavelength of $\lambda$ the direction of the currents generated by the antenna 216 are in the same direction. At wavelengths of $\lambda_{3rd}$ and $\lambda_{5th}$ the direction of the currents generated by the antenna 216 changes with each half-cycle of the waveform. More specifically, at a wavelength of $\lambda_{3rd}$ the direction of the current changes at 310a and 310b and at a wavelength of $\lambda_{5th}$ the direction of the current changes at 320a, 320b, 320c and 320d.

As will be discussed in greater detail hereinbelow, combining microwave waveforms with wavelengths of $\lambda$, $\lambda_{3rd}$ and $\lambda_{5th}$ results in the generation of new waveforms, and resultant electromagnetic fields and current densities. The new waveforms include areas where the two waveforms are additive thereby creating a synergistic effect resulting higher current density and greater tissue penetration. The new waveforms may also include areas where one waveform cancels at least a portion of the second waveform thereby creating areas of reduced energy delivery.

For example, between the proximal end of the proximal radiating portion 216b and position 320a on the antenna 216, the current generated by the three waveforms of $\lambda$ 300, $\lambda_{3rd}$ 310, and $\lambda_{5th}$ 320 is in the same direction, therefore combining any of the waveforms in this portion of the antenna 216 produces an additive ef-

fect. Between position 320a and position 310a the waveforms for $\lambda$ and $\lambda_{3rd}$ are additive to each other and opposite in direction of waveform $\lambda_{5th}$. Therefore, between position 320a and position 310a waveforms $\lambda$ and $\lambda_{3rd}$ are additive if combined and opposite in the direction of waveform $\lambda_{5th}$ and therefore would have a canceling effect. Between position 310a and position 320b the waveforms for $\lambda_{3rd}$ and $\lambda_{5th}$ are additive to each other and opposite in direction of waveform $\lambda$. Between position 310a and position 320b the waveforms $\lambda_{3rd}$ and $\lambda_{5th}$ are additive if combined and opposite in direction of waveform $\lambda$.

Antenna, 216, when driven by a resonant waveform $\lambda$, $\lambda_{3rd}$ or $\lambda_{5th}$, generates an electromagnetic field including "far field" energy that results in a corresponding current density 300, 310 and 320 wherein the current density shape is illustrative of the energy distribution. The ablation region, while related to the shape of the energy distribution, may not resemble the shape of the energy distribution due to dissipation of energy into the surrounding medium before reaching the area defined by the "far-field" region. The "far-field" may correspond to several centimeters outside of the ablation region.

The shape of the "far-field" region and the resulting ablation region may be varied by changing the electromagnetic field during the ablation procedure. For example, changing the waveform between $\lambda$, $\lambda_{3rd}$ or $\lambda_{5th}$ will change the shape of the electromagnetic field and the resulting ablation region.

According to the invention, microwave generator 100 of FIG. 1 simultaneously delivers microwave energy at two different wavelengths to the microwave energy delivery device 110. Microwave generator 100 may select any one of the wavelengths to be delivered to the microwave energy delivery device 110, the intermixing ratio between the two frequencies (e.g., the ratio of energy supplied at each frequency) and the phase relationship, or phase angle, between the one or more signals.

The wavelengths may be selected to create a desirable resultant current density pattern, desirable ablation pattern and/or desirable ablation region or shape. The resultant current density pattern may result in deep penetration of energy into the surrounding medium or may produce an ablation region with a desirable shape or volume.

As will be discussed hereinbelow, combining microwave signals with different wavelengths results in the generation of varying current densities and patterns. For example, combining wavelengths of $\lambda$ and $\lambda_{3rd}$ may produce a current density pattern that provides deep penetration of energy into the surrounding medium at the proximal and distal ends of the antenna 216 and combining wavelengths of combining wavelengths of $\lambda$ and $\lambda_{5th}$ may produce a current density pattern that provides energy delivery to nodes at the midpoint and endpoints of the antenna 216. The clinician may select wavelengths that generate current density patterns that match the target region or area.

In another embodiment, at least one property of a microwave signal is selected to create a desirable current density pattern and/or resulting ablation region. The property may be the phase angle between the two microwave signals, the intensity ratio between the two signals, the energy delivered to the surrounding medium or any other suitable property. For example, the current density pattern created by combining microwave signals with wavelengths of $\lambda$ and $\lambda_{3rd}$ and in phase results in a current density pattern that provides a concentration of current at the proximal and distal ends of the antenna 216 while combining microwave signals with wavelengths of $\lambda$ and $\lambda_{3rd}$ 180° out of phase results in concentration of current at the midpoint with smaller nodes at the proximal and distal ends of the antenna 216.

In yet another embodiment of the present disclosure, at least one property of a microwave signal is adjusted to change the shape of a current density pattern created by combining the microwave signals. For example, the current density pattern may be changed by shifting the phase relationship between the two signals from in phase to 180° out-of-phase or therebetween in phase and -180° out-of-phase. The phase relationship between the two signals may be initially selected to generate a desirable current density pattern and changed during an ablation procedure or during the delivery of energy in order to adjust the current density pattern, increase the size of ablation region or to adjust the shape of the ablation region.

In yet another embodiment of the present disclosure, the property may relate to the intermixing ratio between the two microwave signals. The intermixing ratio between the first and second microwave signals may be initially set in a range from of 99:1 to 1:99. Alternatively, the intermixing ratio may be adjusted during the delivery of microwave energy to change the current density pattern or to change the overall shape and/or volume of the ablation region. For example, during ablation the intermixing ratio may be adjusted to increase the energy delivered at a wavelength less susceptible to changes in the physical properties of the surrounding medium.

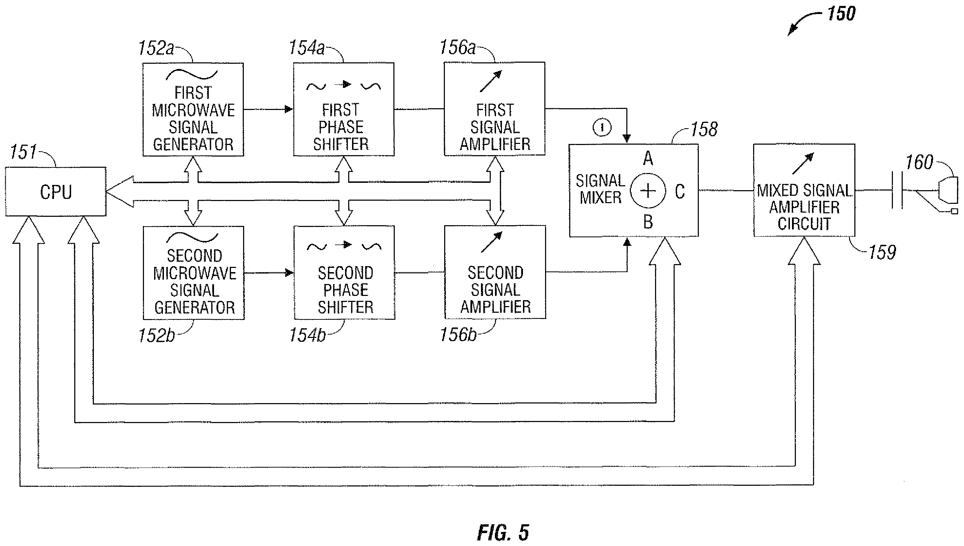

FIG. 5 is a block diagram of the microwave energy generation circuit 150 of FIG. 1 for generating and combining microwave energy at two different wavelengths, according to the present invention. Microwave energy generation circuit 150 includes first and second microwave signal generators 152a, 152b, first and second phase shifters 154a, 154b, first and second signal amplifiers 156a, 156b, signal mixer 158 and a mixed signal amplifier circuit 159. Microwave energy generators and microwave generation circuits are generally known in the art, therefore, only those elements of a microwave energy generation circuit 150 specific to the present disclosure will be described in detail.

First and second microwave signal generators 152a, 152b generate microwave signals at two wavelengths. For example, first microwave signal generator 152a may produce a signal with a wavelength of $\lambda$ and second microwave signal generator 152b may producing a signal with a wavelength that is not equal or equivalent to $\lambda$. In another embodiment, first microwave signal generator 152a produces a signal at a resonant frequency of an antenna and the second microwave signal generator 152b may generate a signal at a harmonic of the resonant frequency.

According to the invention, processor 151 monitors and/or controls the wavelength of the signal produced by first and second signal generators 152a, 152b. Processor 151 may provide one or more parameters, such as a first wavelength of $\lambda$ to the first signal generator 152a and a parameter, such as a second wavelength to the second signal generator 152b. One or more wavelengths are calculated by the processor 151, determined by a parameter of the microwave energy delivery device.

A second wavelength may be a harmonic of the first wavelength $\lambda$, such as, for example, the third harmonic $\lambda_{3rd}$ or fifth harmonic $\lambda_{5th}$. Processor 151 may receive the first wavelength from a clinician and/or may calculate the $\lambda_{3rd}$ or $\lambda_{5th}$ therefrom.

According to the invention, processor 151 includes an algorithm that calculates or adjusts the wavelength generated by first and/or second signal generators 152a, 152b. Mixed signal amplifier circuit 159 provides feedback (i.e., forward power and/or reflected power) to the processor 151 and processor 151 calculates or adjusts one or more properties of the generated signals. For example, processor 151 determines a first resonant frequency by varying the wavelength $\lambda$ generated by the first microwave signal generator 152. The wavelength of the second microwave signal generator 153b is determined by a similar algorithm or may be calculated as a harmonic of the first wavelength, such as, for example $\lambda_{3rd}$ and $\lambda_{5th}$.

Signals from the first and second microwave signal generators 152a, 152b is shifted in phase relative to each other. In one embodiment, microwave energy generation circuit 150 includes first and second phase shifters 154a, 154b for delaying at least one signal thereby changing the phase angle between the signals from the first and second microwave signal generators 152a, 152b, i.e., phase-shifting the signals. The magnitude of the phase-shift between the signals from the first and second microwave signal generators 152a, 152b may be fixed, such as, for example, 180° apart, or processor 151 may dynamically adjust the amount of the phase-shift between the signals. Phase-shifting may be adjusted to vary the current density as described hereinbelow or to maintain resonance with the antenna (not explicitly shown).

Ideally, first and second phase shifters 154a, 154b provide low insertion loss, high power handling and instantaneous phase change response. Signal loss due to the first and second phase shifters 154a, 154b may be overcome by the amount of signal amplification of the first or second signal amplifiers 156a, 156b. First and second phase shifters 154a, 154b may be a switched line phase shifter, a loaded-line phase shifter, a ferroelectric phase shifter, a reflective phase shifter or any other suitable device that shifts the phase of a first microwave signal relative to a second microwave signal.

First and second phase shifters 154a, 154b may be analog or digital and be controlled electrically, magnetically or mechanically. Analog phase shifters may provide variable phase shifting, such as, for example, a variable voltage that may be adjusted through hardware or electronically controlled. Alternatively, analog phase shifters 154a 154b may be controlled by capacitance such as, for example, a nonlinear dielectric such as barium strontium titanate, or a ferroelectric material.

In one embodiment, one or more phase shifters 154a, 154b may be a mechanically-controlled analog phase shifter constructed by selecting a mechanically lengthened the transmission path. Phase shifters 154a, 154b may be configured to lengthen a transmission path or may provide a plurality of transmission paths of varying length and be configured to select one of the plurality of transmission paths that provides the desired phase shift.

In yet another embodiment of the present disclosure, the first microwave signal generator 152a may generate and supply a signal directly to the first signal amplifier 156a thereby bypassing and eliminating the first phase shifter 154a. The signal from the second microwave signal generator 152b may be phase shifted by the second phase shifter 154b relative to the signal from the first microwave signal generator 152a. A second signal amplifier 156b may amplify the single from the second phase shifter 154b to account for any signal loss in the second phase shifter 154b.

First and second signal amplifiers 156a, 156b amplify the signals generated by the respective first and second microwave signal generators 152a, 152b. Signal amplification by the first and second signal amplifiers 156a, 156b may be performed prior to mixing or combining of the two signals by the signal mixer 158, to provide a suitable intermixing ratio between the two signals, as discussed hereinbelow.

Processor 151 may adjust the intermixing ratio between the two signals to provide a desirable current density pattern. For example, processor 151 may initially provide a 3:1 intermixing ratio between the energy delivered at a first wavelength, $\lambda$, to a second wavelength, $\lambda_{3rd}$. As the medium heats and the impendence of the medium increases, the intermixing ratio may be adjusted to a second intermixing ratio, such as, for example, an intermixing ratio of 1:1, 1:3 or 1:99. The adjustment of the intermixing ratio may be changed stepwise or continuously.

The intermixing ratio may be changed dynamically by the processor or the change may be changed based on feedback from the microwave generation circuit 150. In another embodiment the change in the intermixing ratio may be automatically performed by a hardwired circuit (not shown). Alternatively, the change may be initiated or selected by a clinician.

The signals generated from the first and second signal generators 152a, 152b are combined by the signal mixer 158 and amplified by the mixed signal amplifier circuit 159. Signal mixer 158 receives a first signal, generated by the first microwave signal generator 152a on Port A and a second signal, generated by the second microwave signal generator 152b on Port B. Signal mixer 158 combines the signals received on Ports A and Port B and supplies the combined signal to the mixed signal amplifier circuit 159 through Port C. Signal mixer 158 may provide suitable isolation between Ports A and B while keeping the signals from Port A and Port be in phase (0° difference). Port A, Port B and/or Port C may provide 50 ohm nominal impedance or any other suitable or desirable impedance.

Amplification of the mixed signal provided from Port C of the signal mixer 158 is performed by the mixed signal amplifier circuit 158. The amount of amplification, or the power level of the signal delivered to the delivery device port 160, is determined by the processor 151 or selected or entered by a clinician via the front panel 105 of the microwave generator of FIG. 1. Alternatively, the power level of the signal delivered to the delivery device port 160 may be determined, calculated or selected by the microwave generator based on an energy delivery parameter or the procedure performed.

Mixed signal amplifier circuit 150 typically includes one or more amplifiers including a power amplifier, a circulator or other suitable means of signal isolation and includes a dual directional coupler or other means to measure forward and/or reflective power or any other suitable signal measurement device.

The system and methods disclosed herein may be used in conjunction with other tissue or energy measurement systems and techniques, such as, for example, tissue impedance measuring, tissue temperature measuring, current, voltage, power and energy measuring and phase of voltage and current measuring.

The method disclosed herein may be carried out using a feedback system incorporated into an electrosurgical system or may be a stand-alone modular embodiment (e.g., removable modular circuit configured to be electrically coupled to various components, such as a generator, of the electrosurgical system).

*Combined Frequency Waveforms and Current Density Patterns*

The current density patterns produced by an antenna when the antenna is driven with various resonant frequencies harmonics form different patterns of energy deposition along the antenna's length. The antenna, when driven with energy at a single frequency, will form "hot spots" at one or more points along the antenna or in the surrounding medium wherein the location of the "hot spots" is related to a relative maximum or a maximum current density. The "hot spots" locations are dependant on whether the antenna is driven with a first, third or fifth order harmonic, generally fixed in location and may be predictable and/or calculated with a suitable computer simulation model.

A resultant current density waveform, formed by combining two or more waveforms, produces a resultant current density waveform current density pattern that is transient (i.e., the current density changes based on a relationship between the two or more waveforms at any point in time). As such, the position of the maximum current density and the position of "hot spots" related to the maximum current density may not be constant and may change based on this relationship between the two or more waves. In addition, the resultant current density waveform and related current density pattern may be non-monotonic with a plurality of relative maximum current density maximums thereby resulting in a plurality of "hot spots", each related to a relative maximum or maximum current density.

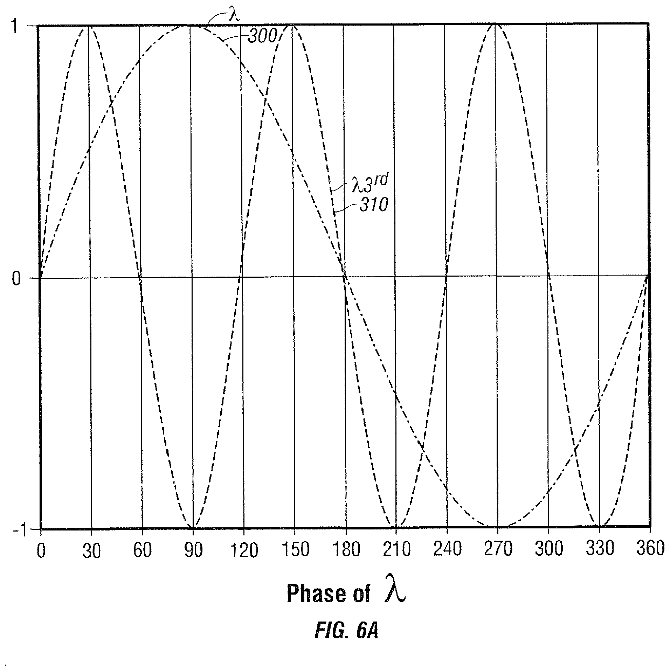

FIGS. 6A-6B further illustrates the changing current density pattern of the resultant current density waveform 330a-1. FIG. 6A is a plot of two waveforms, $\lambda$ 300 and $\lambda_{3th}$ 310, wherein $\lambda_{3th}$ 310 is the third harmonic of $\lambda$ 300 and $\lambda$ 300 and $\lambda_{3rd}$ 310 are "in phase" with each other. In the plot, time is represented in the 'phase angle' of the $\lambda$ 300 waveform and the magnitude is normalized.

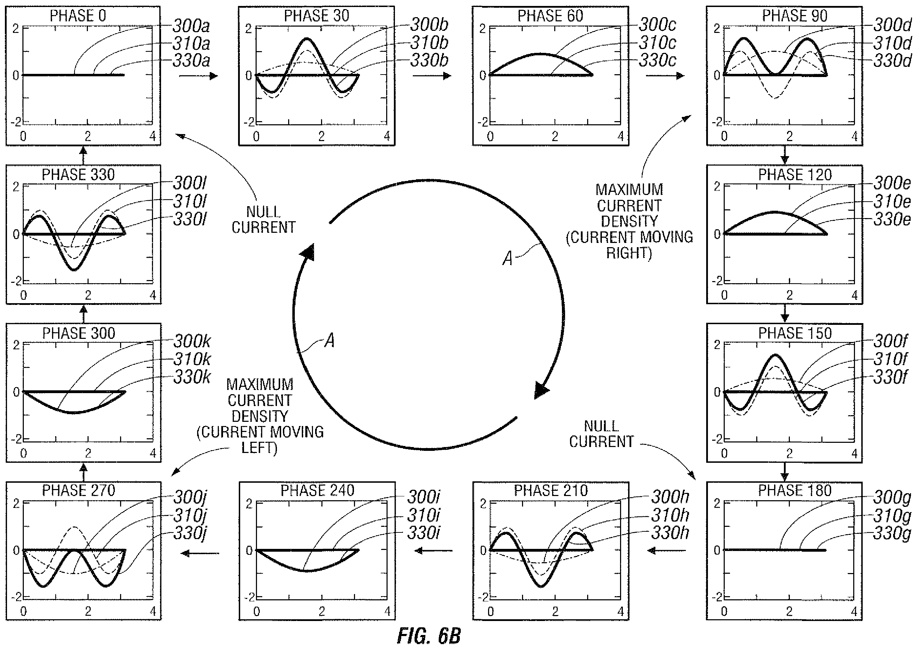

FIG. 6 illustrates the two waveforms $\lambda$ 300and $\lambda_{3th}$ 310 from FIG. 6A and the current density pattern of the resultant current density waveform 330a-1 at a plurality of instantaneous points in time. The time interval is shifted by 30° thereby illustrating the change in the instantaneous current density pattern of the two waveforms 300a-1, 310a-1 and the resultant current density waveform 330a-1. For each plot in FIG. 6B the antenna "feedpoint" can be envisioned at the x=1.5. Plots are normalized to the resonant frequency such that the 'ends' of the antenna are at 0 and 3. The current at the antenna 'ends' are idealized with a magnitude of zero. While the present embodiment illustrates a balanced dipole antenna the embodiments illustrated herewithin may be used with other types of antennas, such as, for example, an unbalanced dipole antenna.

At 0° (PHASE 0) both waveforms, $\lambda$ 300a and $\lambda_{3th}$ 310a exhibit 'null' current along the antenna, resulting in a resultant current density waveform 330a also exhibiting 'null' current along the antenna.

At 30° (PHASE 30) the resultant current density waveform 330b generated from $\lambda$ 300b and $\lambda_{3th}$ 310b produces three relative current density maximums. The waveform $\lambda_{3th}$ 310b is at its peak (at the feed point), the waveform $\lambda$ 300b is half of its peak with the current distributed across the dipole length.

At 60° (PHASE 60) waveform $\lambda_{3th}$ 310c exhibits 'null' current along the antenna and the resultant current density waveform 330c is equal to waveform $\lambda$ 300c.

At 90° (PHASE 90) the resultant current density waveform 330d generated from waveforms $\lambda$ 300d and $\lambda_{3th}$ 310d produces two relative current density maximums. The direction of current flow for both relative current density maximums is to the right. Both waveforms $\lambda$ 300d and $\lambda_{3th}$ 310d are at a peak, 180° out of phase thereby canceling at the feed point but not canceling along the dipole away from the feed point.

At 120° (PHASE 120) waveform $\lambda_{3th}$ 310e exhibits 'null' current along the antenna and the resultant current density waveform 330e is equal to waveform $\lambda$ 300e.

At 150° (PHASE 150) the resultant current density waveform 330f generated from $\lambda$ 300f and $\lambda_{3th}$ 310f produces three relative current density maximums.

At 180° (PHASE 180) both waveforms, $\lambda$ 300g and $\lambda_{3th}$ 310g exhibit 'null' current along the antenna, resulting in a resultant current density waveform 330g also exhibiting 'null' current along the antenna.

At 210° (PHASE 210) the resultant current density waveform 330h generated from $\lambda$ 300h and $\lambda_{3th}$ 310h produces three relative current density maximums.

At 240° (PHASE 240) waveforms $\lambda_{3th}$ 310i exhibits 'null' current along the antenna and the resultant current density waveform 330i is equal to waveform $\lambda$ 300i.

At 270° (PHASE 270) the resultant current density waveform 330j generated from waveforms $\lambda$ 300j and $\lambda_{3th}$ 310j produces two relative current density maximums. The direction of current flow for both relative current density maximums is to the left.

At 300° (PHASE 300) waveform $\lambda_{3th}$ 310k exhibits 'null' current along the antenna and the resultant current density waveform 330k is equal to waveform $\lambda$ 300k.

At 330° (PHASE 330) the waveform $\lambda_{3th}$ 310l exhibits 'null' current along the antenna and the resultant current density waveform 330l is equal to waveform $\lambda$ 300l.

As indicated by Arrows A, the cycle is a repeating cycle with the current density patterns in the PHASE 0 synonymous with a 360° phase angle plot that follows the PHASE 330 plot illustrated in FIG. 6B.

Examining and comparing the current density patterns at various instantaneous points in time across the entire cycle, from 0° to 330° in 30° intervals PHASE 0 to PHASE 330, respectively, illustrates that the current density generated by the antenna when driven by the resultant current density waveform 330a-1 is transient. More specifically, "hot spots", areas of concentrated energy at maximum current density positions, generated by the resultant current density waveforms 330a-1 along the length of the antenna are continuously changing location. For example, in plots PHASE 30, PHASE 150, PHASE 210 and PHASE 330 the maximum current density is adjacent the feed point of the antenna with relative maximum current densities adjacent the ends of the antenna and in the plots PHASE 90 and PHASE 270 the antenna exhibits null current at the feed point.

In addition, as illustrated in FIGS. 6B, at various 30° intervals the number of relative maximum current density regions, and the number of related "hot spots", is not constant. For example, the resultant current density waveform 330a-1 generates three relative or maximum current density regions in the plots PHASE 30, PHASE 90, PHASE 150, PHASE 210, PHASE 270 and PHASE 330. The resultant current density waveform 330a-1 only generates one relative maximum current density region in the plots PHASE 60, PHASE 120, PHASE 240 and PHASE 300 and no current density regions when the resultant current density waveforms exhibits 'null' current along the antenna as illustrated in the plots PHASE 0 and PHASE 180.

As such, the current density pattern of the resultant current density waveform 330a-1 changes in shape and magnitude during the waveform cycles based on the relationship between the two or more waveforms 300a-1, 310a-1 that combine and form the resultant current density waveform 330a-1. The constantly changing current density results in a varying distribution and disbursement of energy into the surrounding medium. As such, the ablation region generated in the surrounding medium by the antenna driven with the resultant current density waveform 330a-1 will form a shape related to this transient current density. The shape of the ablation region may exhibit features from the maximum current density regions and the relative maximum current density regions described hereinabove.

As a result of the shifting position of the maximum current density along the antenna regions, the ablation region produced in the surrounding medium by the antenna may exhibit an irregular shape. The ablation region may exhibit features that resemble features from one or more of the plots illustrated in FIG. 6B. For example, the ablation region may include one or more features adjacent the ends of the antenna generated by the relative current density regions illustrated in the plots PHASE 30, PHASE 90, PHASE 150, PHASE 210, PHASE 270 and PHASE 330. The ablation region may include one or more features between the proximal and distal ends of the antenna and one or more features between the relative current density regions adjacent the feedpoint (or middle portion of the antenna) as illustrated in the plots PHASE 30, PHASE 60, PHASE 120, PHASE 150, PHASE 210, PHASE 240, PHASE 300 and PHASE 330.

In another embodiment of the present disclosure, an approximation of the ablation region formed by the resultant current density waveforms 330a-1 may be estimated by an algorithm that determines the energy contribution to the surrounding medium at several instantaneous points in time of the resultant current density waveform 330a-1. The magnitude of the current density of the resultant current density waveform 300a-1 is a maximum at PHASE 90 and PHASE 270, therefore, the energy contributed to the surrounding medium may be a maximum during this time. This may result in the general overall shape of the ablation region approximated by the algorithm to be related to the current density patterns at PHASE 90 and PHASE 270. The ablation region approximated by the algorithm may include additional features generated during the various other phase angles, such as, for example, an additional feature adjacent the feed point (or middle of the antenna) may be generated by the various other current density patterns at different phase angles.

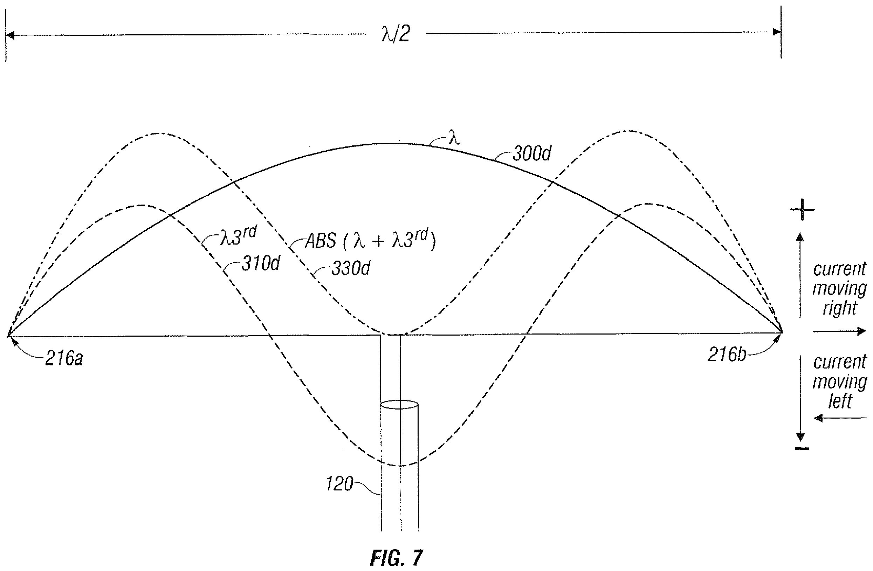

FIG. 7 is a further illustration of the instantaneous current density patterns from FIG. 6B with the half-wave di-pole antenna 216 of FIG. 3 driven by the microwave energy generation circuit 150 of FIG. 5. The half-wave di-pole antenna 216 may have a physical length of approximately $\lambda/2$ or another suitable length that produces resonance. FIG. 7 includes a graphical representation of the instantaneous maximum current density along the antenna 216 for the microwave signal from FIG. 6A with a wavelengths of $\lambda$ 300, the microwave signal with a wavelength of $\lambda_{3th}$ 310 and a resultant current density waveform 330d formed by combining the microwave signals with wavelengths of $\lambda$ 300 and $\lambda_{3th}$ 310.

The $\lambda$ current density 300d is illustrative of the current density generated in the surrounding medium by an antenna when driven by the microwave waveform provided to Port A of the signal mixer 158 of FIG. 5. Similarly, the $\lambda_{3th}$ current density 310d is illustrative of the current density generated in the surrounding medium by an antenna when driven by the microwave waveform provided to Port B of the signal mixer 158 of FIG. 5. The resultant current density waveform, provided on Port C of the signal mixer 158 of FIG. 5, is the sum of the $\lambda$ 300d and $\lambda_{3th}$ 310d waveforms, is generated by the signal mixer 158 and provided to Port C. In FIG. 7, the maximum instantaneous current densities 300d, 310d and 330d, generated by driving the microwave antenna with the various waveforms, are generally scaled to approximate the relative intensity level between the microwave waveforms.

The waveforms at Ports A and B are in phase and have a 1:1 intensity level. The magnitude of the resultant current density of the waveform at Port C is shown as 330d and is equal to:

$$\text{Abs}\,(\lambda + \lambda_{3rd})$$

More specifically, the waveforms in FIG. 7 illustrate the current density of each waveform at the instantaneous point in time when the waveforms $\lambda$ 300 and waveform $\lambda_{3th}$ 310 are a maximum and is a further illustration of the plot PHASE 90 from FIG. 7B. The resultant current density waveform 300d is a maximum at a point proximal to the midpoint of the proximal radiating portion 216a and a point distal to the midpoint of the distal radiating portion 216b. The current density is a minimum at the distal and proximal ends of the antenna 216 and at the midpoint of the antenna 216.

While the instantaneous resultant current density waveform 330d in FIG. 7 may be a maximum, as illustrated in FIGS. 6A-6B and discussed hereinabove, the maximum resultant current density is only generated at a single instantaneous point in time and the resultant current density waveform exhibits many additional shapes over time thereby resulting in an ablation region that may resemble the maximum resultant current density waveform 330d but also exhibits may other features. As such, the current densities and instantaneous current densities illustrated herewithin are provided to demonstrate the synergistic affect of combining two or more microwave frequencies. The current density generated from the resultant current density waveforms may reflect a general shape of a potential ablation region when the energy delivered to tissue is at a maximum. The actual size and shape of an ablation region formed from an antenna producing one of the current densities illustrated herewithin is dependant on several other factors including, but not limited to, the varying current density of the resultant current density waveform, the properties of the surrounding medium, the rate of energy delivery to the medium and the total energy delivered to the medium. In addition, the figures contained herein provide a comparison between the current density generated by a signal with energy at a single microwave frequency and the current density of a resultant current density waveform generated from combining two or more microwave frequencies.

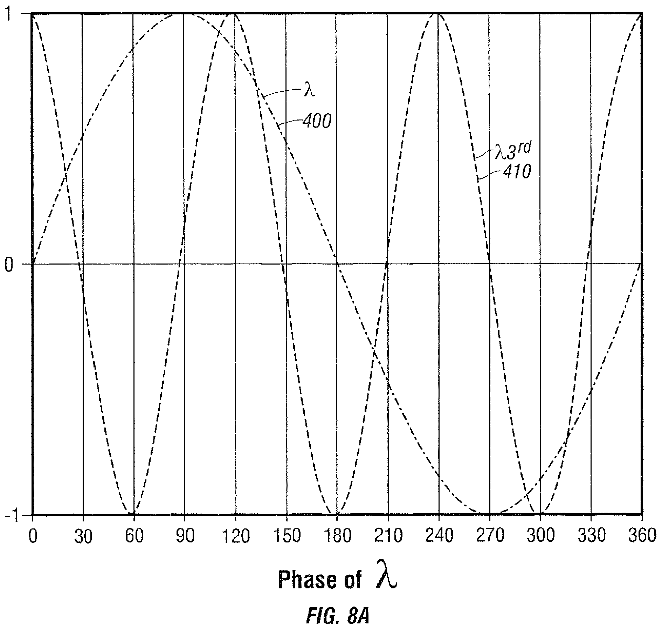

In yet another embodiment of the present disclosure one or more of the microwave signals that are combined to generate the combined microwave signal are shifted in phase with respect to each other. For example, FIG. 8A is a plot of two waveforms, $\lambda$ 400 and $\lambda_{3rd}$ 410, wherein $\lambda_{3rd}$ 410 is the third harmonic of $\lambda$ 400 and $\lambda$ 400 and $\lambda_{3rd}$ 410 are out of phase by with respect to each other. Waveform $\lambda$ 400 leads waveform $\lambda_{3rd}$ 410 by 30° with respect to the $\lambda$ 400 time scale (or a 90° lag with respect to a $\lambda_{3rd}$ 410 time scale). In the plot, time is represented in the time scale or 'phase angle' of the $\lambda$ 400 waveform and the magnitude of the waveforms are normalized.

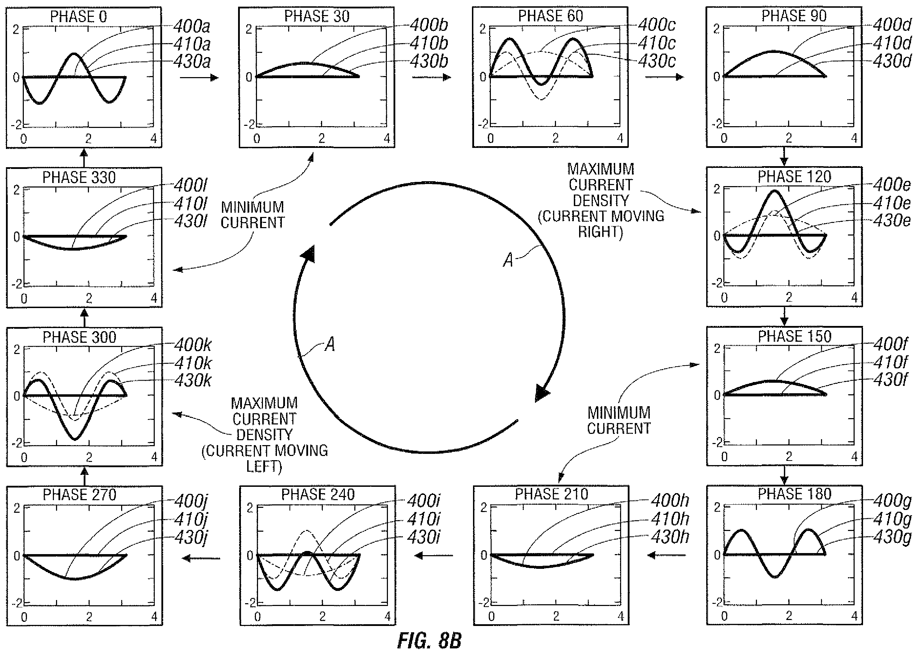

FIG. 8B illustrates the two waveforms $\lambda$ 400 and $\lambda_{3th}$ 410 from FIG. 8A and the current density pattern of the resultant current density waveform 430a-1 at a plurality of instantaneous points in time. The time interval is shifted by 30° thereby illustrating the change in the instantaneous current density patterns of the two waveforms 400a-1, 410a-1 and the resultant current density pattern waveform 430a-1. For each plot in FIG. 8B the antenna "feedpoint" can be envisioned at the x=1.5. Plots are normalized to the resonant frequency such that the 'ends' of the antenna are at 0 and 3. The current at the antenna 'ends' are idealized at 0 magnitudes. While the present embodiment illustrates a balanced dipole antenna the embodiments illustrated herewithin may be used with other types of antennas, such as, for example, an unbalanced dipole antenna and.

At 0° (PHASE 0) waveforms $\lambda$ 400a exhibits 'null' current along the antenna and waveform $\lambda_{3rd}$ 410a is a maximum. The resultant current density waveform 430a is equal to the current density pattern generated by the waveform $\lambda_{3rd}$ 410a.

At 30° (PHASE 30) waveform $\lambda_{3rd}$ 410b exhibits 'null' current along the antenna and waveform $\lambda$ 400b is growing to about half of the absolute peak. The resultant current density waveform 430b is distributed sinusoidally along the dipole.

At 60° (PHASE 60) waveform $\lambda_{3th}$ 410c is a maximum and waveform $\lambda$ 400c is approaching a maximum in the opposite direction. The current density generated for waveform $\lambda$ 400c and waveform $\lambda_{3th}$ 310c are additive at the ends of the antenna and cancel near the feedpoint. As such, the resulting current density pattern 430c is greater at the ends of the antenna with little current at the feedpoint.

At 90° (PHASE 90) waveform $\lambda_{3th}$ 410d exhibits 'null' current along the antenna and the resultant current density waveform 430d is equal to waveform $\lambda$ 400d, which is a maximum and distributed sinusoidally along the dipole,

At 120° (PHASE 120) waveform $\lambda_{3th}$ 410e is a maximum and waveform $\lambda$ 400e is slightly less than a maximum. The resultant current density waveform 430e is a maximum at the feedpoint and greater than waveforms $\lambda$ 400e and $\lambda_{3th}$ 410e individually.

At 150° (PHASE 150) waveform $\lambda_{3th}$ 410f exhibits 'null' current along the antenna and the resultant current density waveform 430f is equal to waveform $\lambda$ 400f, which is decaying to less than half of the peak, approaching zero and distributed sinusoidally along the dipole.

At 180° (PHASE 180) waveform, $\lambda$ 400g exhibits 'null' current along the antenna and the resultant current density waveform 400g is equal to waveform $\lambda_{3th}$ 410g, which is at a maximum thereby forming current density peaks at the feedpoint and the distal and proximal ends of the antenna.

At 210° (PHASE 210) waveform $\lambda_{3th}$ 410h exhibits 'null' current along the antenna and the resultant current density waveform 430h is equal to waveform $\lambda$ 400h, which growing to a maximum in the opposite direction and distributed sinusoidally along the dipole.

At 240° (PHASE 240) waveform $\lambda_{3th}$ 410i is a maximum and waveform $\lambda$ 400i is approaching a maximum in the opposite direction. The current density generated for waveform $\lambda$ 400i and waveform $\lambda_{3th}$ 410i are additive at the ends of the antenna and are opposite at the feedpoint. The resulting current density pattern 430i is greater at the ends of the antenna with little current at the feedpoint.

At 270° (PHASE 270) waveform $\lambda_{3th}$ 410j exhibits 'null' current along the antenna and the resultant current density waveform 430j is equal to waveform $\lambda$ 400j, which is a maximum and distributed sinusoidally along the dipole.

At 300° (PHASE 300) waveform $\lambda_{3th}$ 410k is a maximum and waveform $\lambda$ 400k is slightly less than a maximum. The resultant current density waveform 430k is a maximum at the feedpoint and greater than waveforms $\lambda$ 400k and $\lambda_{3th}$ 410k individually.

At 330° (PHASE 0) waveform $\lambda_{3th}$ 4101 exhibits 'null' current along the antenna and the resultant current density waveform 4301 is equal to waveform $\lambda$ 4001.

As indicated by Arrows A, the cycle is a repeating cycle with the current density patterns in the PHASE 0 plot synonymous with a 360° phase angle plot that would follow the PHASE 330 plot illustrated in FIG. 8B.

Examining and comparing the current density patterns at various points in time across the entire cycle, from 0° to 330° (PHASE 0 TO PHASE 330, respectively), illustrates that the resultant current density waveforms 330a-1 generated by the antenna when driven by the two microwave frequencies waveforms is transient. More specifically, in the resultant current density waveform 430a-1 the location of potential "hot spots", or the position of maximum current densities points along the length of the antenna, are always changing location. For example, in PHASE 120 and PHASE 300 of FIG. 8B the maximum current density is adjacent the feed point of the antenna and in PHASE 60 and PHASE 240 the maximum current density is adjacent the distal and proximal ends of the antenna.

In addition, as illustrated in FIG. 8B, at various 30° intervals the number of relative maximum current density regions, and the number of related "hot spots", changes. For example, resultant current density waveform 430a-1 generates three relative maximum current density regions at a phase angles of 0° (PHASE 0), one maximum current density region at a phase angle of 30° (PHASE 30) and two relative maximum current density regions at a phase angle of 60° (PHASE 60). The resultant current density waveforms 430a-1 generate one relative maximum current density region in the plots at phase angles of 30°, 90°, 150°, 210°, 270° and 330° PHASE 30, PHASE 90, PHASE 150, PHASE 210, PHASE 300°, respectively.

With reference to FIGS. 6A and 8A shifting the phase of one waveform relative to the other waveform results in a very different resultant current density patterns as illustrated in FIGS. 6B and 8B. As such, the resultant current density waveforms 330a-1, 430a-1 are dependant on the properties of the two waveforms 300 and 310, 400 and 410, respectively, (i.e., frequency, magnitude, energy content) and the phase relationship between the two waveforms 300 and 310, 400 and 410, respectively.

A comparison between the resultant current density waveforms 330a-1 in FIG. 6B and the resultant current density waveforms 440a-1 in FIG. 8B illustrates the effect of phase shifting the waveforms 300, 310, 400, 410 relative to each other. For example, in 6A and 8A waveform $\lambda$ 300 are an absolute maximum at 90° and 270°. In FIG 6A the relative maximums for waveform $\lambda_{3th}$ 300 are in phase with the relative maximums of waveform $\lambda$ 330 thereby resulting in a unique set of instantaneous current densities illustrated in FIG. 6B. In contrast, in FIG. 8B the relative maximums for waveform $\lambda_{3th}$ 400 are not phase with the relative maximums of waveform $\lambda$ 430

thereby resulting in the unique set of instantaneous current densities as illustrated in FIG. 8B. In each example, the two waveforms 300, 310 and 400, 410 that are combined to generate the resultant current density waveforms are identical in frequency in magnitude but phase-shifted relative to each other. This phase shift results in a resultant current density waveform 330a-1, 430a-1 with new and unique features and may result in an ablation region related to the new and unique features of the resultant current density waveforms 330a-1, 430a-1 (i.e., the ablation region generated in the surrounding medium by the antenna driven with the resultant current density waveform 330a-1, 430a-1 will form a shape related to this transient current density, therefore the shape of the ablation region may exhibit features from each current density regions).

As a result of the shifting position of the maximum current density along the antenna regions over time, the ablation region produced in the surrounding medium by the antenna will be irregularly shaped. The ablation region may exhibit features that resemble features from one or more of the plots illustrated in FIG. 6B. For example, the ablation region may include one or more features adjacent the ends of the antenna generated by the relative current density regions illustrated in the plots PHASE 0, PHASE 60, PHASE 120, PHASE 180, PHASE 240 and PHASE 300. The ablation region may include one or more features between the proximal and distal ends of the antenna and one or more features between the relative current density regions adjacent the feedpoint as illustrated in the plots PHASE 0, PHASE 120, PHASE 180 and PHASE 300.

Alternatively, an approximation of the ablation region formed by the resultant current density waveform 400a-1 may be estimated by the energy contribution during each phase of the resultant current density waveform. The magnitude of the current density of the resultant current density waveform is a maximum at phase angles of 120° and 300° (see PHASE 120, PHASE 300), therefore, the energy contributed to the surrounding medium may be at a maximum during this instantaneous point in time. This may result in the general overall shape of the ablation region to be more related to the current density patterns at these phase angles. The ablation region may include additional features generated during the various other phase angles, as discussed hereinabove.

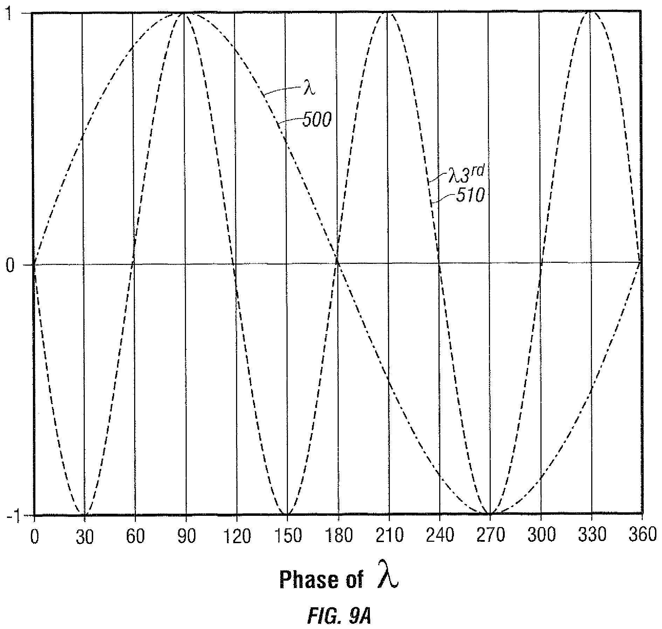

In yet another embodiment of the present disclosure, the phase-shift between the microwave signals may produce a resultant current density waveform that provides deep penetration of energy into tissue. For example, FIG. 9A is a plot of two waveforms, $\lambda$ 500 and $\lambda_{3th}$ 510, wherein $\lambda_{3th}$ 510 is the third harmonic of $\lambda$ 500 and $\lambda$ 500 and $\lambda_{3th}$ 510 are out of phase by with respect to each other. Waveform $\lambda$ 500 leads waveform $\lambda_{3th}$ 510 by 60° with respect to the $\lambda$ 500 time scale (or a 180° lag with respect to the $\lambda_{3th}$ 510 time scale). In the plot, time is represented in the time scale or 'phase angle' of the $\lambda$ waveform 500 and the magnitude of the waveforms 500, 510 are normalized.

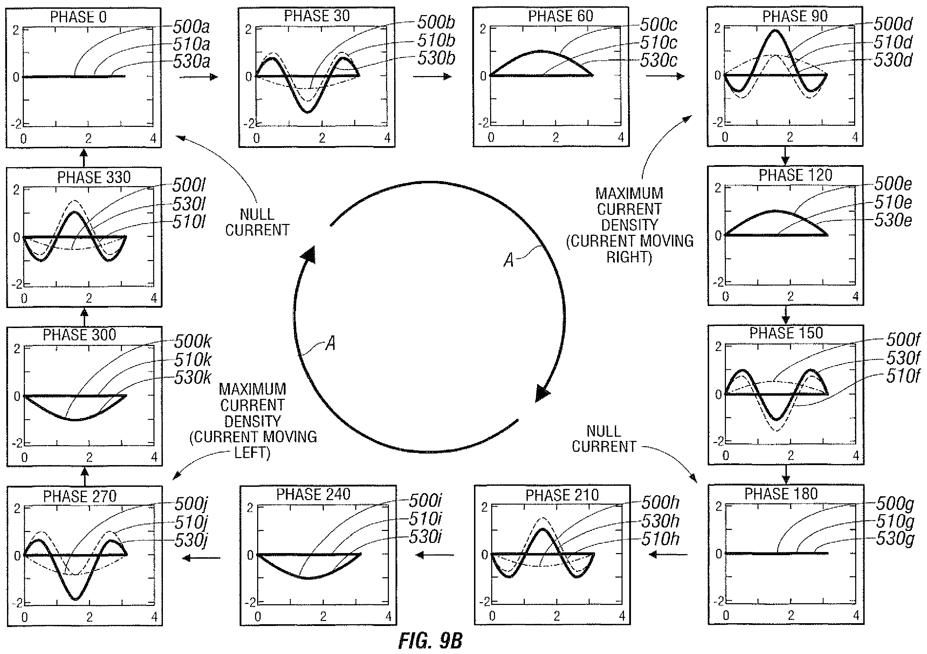

FIG. 9B illustrates the two waveforms λ 500 and λ λ$_{3th}$ 510 from FIG. 9A and the current density pattern of the resultant current density waveform 530a-530l at a plurality of instantaneous points in time. The time interval is shifted by 30° thereby illustrating the change in the instantaneous current density patterns of the two waveforms 500a-500l, 510a-500l and the resultant current density pattern waveform 530a-500l. For each plot in FIG. 9B the antenna "feedpoint" can be envisioned at the x=1.5. Plots are normalized to the resonant frequency such that the 'ends' of the antenna are at 0 and 3. The current at the antenna 'ends' are idealized at 0 magnitudes. While the present embodiment illustrates a balanced dipole antenna the embodiments illustrated herewithin may be used with other types of antennas, such as, for example, an unbalanced dipole antenna.

At 0° (PHASE 0) waveforms λ 500a exhibits 'null' current along the antenna and waveform λ$_{3th}$ 510a is a maximum. The resultant current density waveform 530a is equal to the current density pattern generated by the waveform λ$_{3th}$ 510a.

At 30° (PHASE 30) waveform λ$_{3th}$ 510b is a maximum and waveform λ 500b is growing to about half of the absolute peak in the opposite direction. The resultant current density waveform 530b includes a maximum current density adjacent the antenna feed point and a relative maximum current densities adjacent the ends of the antenna.

At 60° (PHASE 60) waveform λ$_{3th}$ 510c exhibits 'null' current along the antenna and the resultant current density waveform 530c is equal to waveform λ 500c, which is approaching a maximum.

At 90° (PHASE 90) waveform λ 500d and λ$_{3th}$ 510d are a maximum and the resultant current density waveform 530d generates a maximum current density at the feed point equal to about twice the current density of waveforms λ 500d and λ$_{3th}$ 510d individually.

At 120° (PHASE 120) waveform λ$_{3th}$ 510e exhibits 'null' current along the antenna and the resultant current density waveform 530e is equal to waveform λ 500e, which is decreasing from a maximum.

At 150° (PHASE 150) waveform λ$_{3th}$ 510f is a maximum and waveform λ 500f is decreasing and about half of the absolute peak in the opposite direction. The resultant current density waveform 530f includes a maximum current density adjacent each end of the antenna and a relative maximum current density at the antenna feedpoint.

At 180° (PHASE 180) waveforms λ 500g exhibits 'null' current along the antenna and waveform λ$_{3th}$ 510g is a maximum. The resultant current density waveform 530g is equal to the current density pattern generated by the waveform λ$_{3th}$ 510g.

At 210° (PHASE 210) waveform λ$_{3th}$ 510h is a maximum and waveform λ 500h is decreasing and about half of the absolute peak in the opposite direction. The resultant current density waveform 530h includes a maximum current density adjacent each end of the antenna and a relative maximum current density at the antenna feedpoint.

At 240° (PHASE 240) waveform λ$_{3th}$ 510i exhibits 'null' current along the antenna and the resultant current density waveform 530i is equal to waveform λ 500i, which is decreasing to a maximum.

At 270° (PHASE 270) waveforms λ 500j and λ$_{3th}$ 510j are a maximum and the resultant current density waveform 530d generates a maximum current density at the feed point equal to about twice the current density of waveforms λ 500 and λ$_{3th}$ 510d individually. The current flow is equal in magnitude and opposite in of the current generated by the resultant current density waveform 530d in plot PHASE 90.

At 300° (PHASE 300) waveform λ$_{3th}$ 510k exhibits 'null' current along the antenna and the resultant current density waveform 530k is equal to waveform λ 500k, which is decreasing from a maximum.

At 330° (PHASE 0) waveform λ$_{3th}$ 510l is a maximum and waveform λ 500l is decreasing and about half of the absolute peak in the opposite direction. The resultant current density waveform 530l includes a maximum current density adjacent each end of the antenna and a relative maximum current density at the antenna feedpoint.

As indicated by Arrows A, the cycle is a repeating cycle with the current density patterns in the PHASE 0 plot synonymous with a 360° phase angle plot that would follow the PHASE 330 plot illustrated in FIG. 9B.

With reference to FIG. 6A, FIG. 8A and FIG. 9A shifting the phase of one waveform relative to the other waveform may result in deep penetration of energy into tissue. In general, the plots in FIGS. 6A and 8A illustrate current density patterns that provide energy penetration at both the feedpoint and the ends of the antenna while the plots in FIG. 9A provide the greatest energy penetration at the feedpoint. For example, as illustrated in FIG 9A, both waveforms λ 500 and λ$_{3th}$ 510 are at a maximum magnitude in the same direction at 90° and 270°. The current density waveforms plots in FIG. 9B at 90° and 270° PHASE 90, PHASE 270 further illustrate that the resultant current density waveforms 530d, 530j provide maximum energy penetration into tissue.

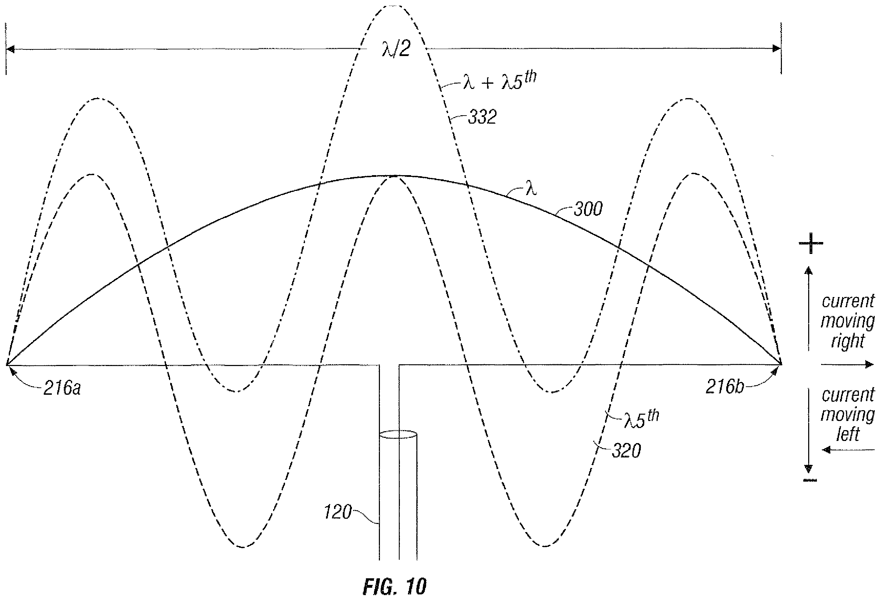

The microwave energy generation circuit 150 of FIG. 5 may combine a first and second microwave signal with various frequency and/or phase combinations to generate a desirable resultant current density waveform as discussed hereinabove. FIGS. 10-16 provide further illustrations of specific frequency and phase combinations. Each of FIGS. 10-16 include plots of the instantaneous current density patterns in which the first and second microwave signals that are combined to generate a resulting waveform are at a maximum. The resultant current density waveform is also an instantaneous current density waveform and is only an exemplary example of the varying current density waveforms as illustrated in FIGS. 6A and 6B, FIGS. 8A and 8B, and FIGS. 9A and 9B.

FIG. 10 illustrates current density plots of a microwave signal with a wavelength of λ 300, supplied to Port A, a microwave signal with a wavelength of $\lambda_{5th}$ 332, supplied to Port B and the resultant current density waveform 332. The waveforms at Ports A and B are in phase and have a 1:1 intensity level. The magnitude of the resultant current density is shown as 336 and is equal to:

$$\text{Abs } (\lambda + \lambda_{5th})$$

The current density is a maximum near the midpoint of the proximal radiating portion 216a and near the midpoint of the distal radiating portion 216b. In addition, a second relative current density maximum is located proximal the distal end of the antenna 216 and distal the proximal end of the antenna 216. The current density is a minimum at the midpoint of the antenna 216, the endpoints of the antenna 216 and approximately midway between each of the maximum current density and the relative maximum current density on both the distal and proximal radiating portions 216a, 216b,

The higher frequency waveform $\lambda_{5th}$ 320 when combined with waveform λ 300, provides a relative maximum current density portion adjacent the proximal end of the proximal antenna portion 216a and the distal end of the distal antenna portion 216b. The higher frequency waveform $\lambda_{5th}$ positions the relative maximum current density portions further to the ends of the antenna.

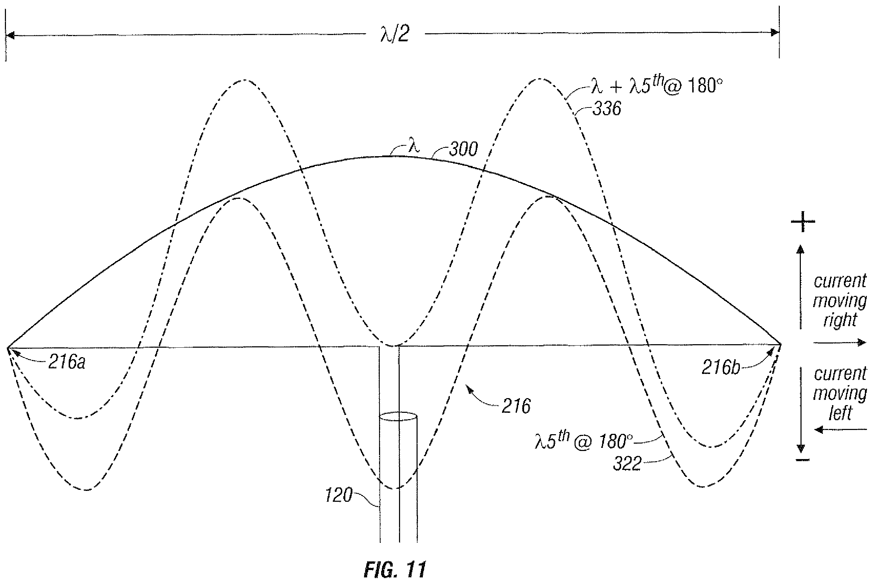

FIG. 11 illustrates current density plots of a microwave signal with a wavelength of λ 300, supplied to Port A, a microwave signal with a wavelength of $\lambda_{5th}$ 322, supplied to Port B and the resultant current density waveform 336. The λ 300 waveform leads the $\lambda_{5th}$ 322 waveform by 36° on the λ phase angle (180° on the $\lambda_{5th}$ phase angle). Ports A and B are shifted in phase by 36 degrees and have a 1:1 intensity level. The magnitude of the resultant current density is shown as 336 and is equal to:

$$\text{Abs } (\lambda + \lambda_{5th} @ 180°)$$

The resultant current density waveform 336 exhibits "null" current at the midpoint with a maximum current density portion and a relative maximum current density portion toward the proximal and distal ends 216a, 216b of the antenna 216.

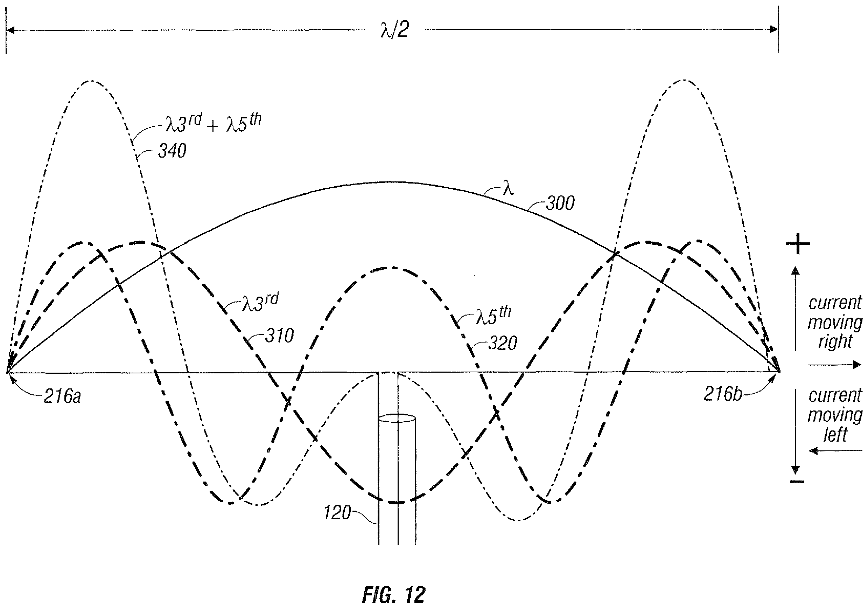

FIG. 12 illustrates current density plots of a microwave signal with a wavelength of $\lambda_{3rd}$ 310, supplied to Port A, a microwave signal with a wavelength of $\lambda_{5th}$ 320 supplied to Port B and the resultant current density waveform 340. The waveforms at Ports A and B are in phase and have a 1:1 intensity level. The current density for a λ 300 waveform is provided for reference. The magnitude of the resultant current density 340 of the waveform at Port C is equal to:

$$\text{Abs } (\lambda_{3rd} + \lambda_{5th})$$

The resultant current density waveform 340 exhibits "null" current at the midpoint with a maximum current density portion adjacent the proximal and distal ends 216a, 216b of the antenna 216. Two relative current density maximums are positioned between the feedpoint and the maximum current density portion. The current density is a minimum at the distal and proximal ends of the antenna 216, the midpoint of the antenna 216 and near the midpoint of the distal and proximal radiating portions 216a, 216b. In general, the current density is focused toward the distal and proximal ends of the antenna 216.

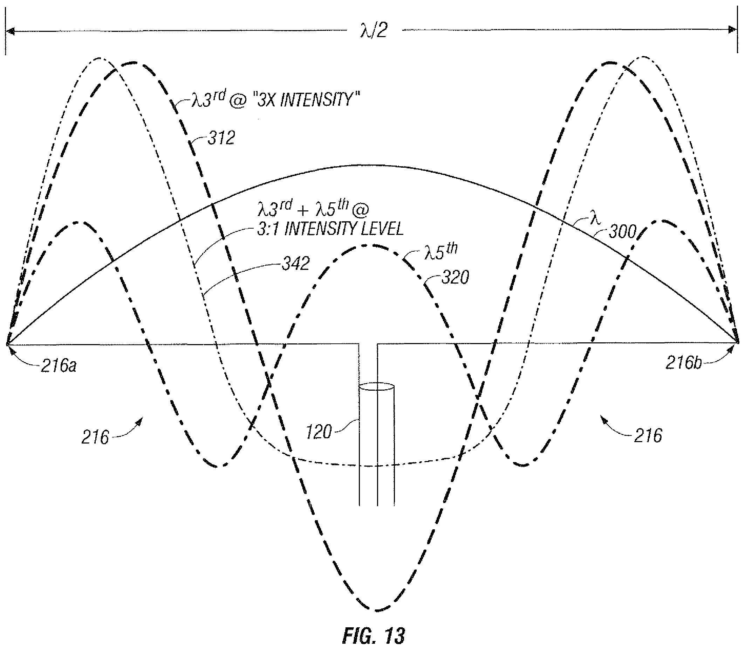

FIG. 13 illustrates current density plots of a microwave signal with a wavelength of $\lambda_{3rd}$ 312, supplied to Port A, a microwave signal with a wavelength of $\lambda_{5th}$ 320 supplied to Port B and the resultant current density waveform 342. The waveforms at Ports A and B are in phase and have a 3:1 intensity level. A microwave waveform with a wavelength of λ 300 is provided for reference. The magnitude of the resultant current density of the waveform at Port C is shown as 342 and is equal to:

$$\text{Abs } [(\lambda_{3rd} * 3) + \lambda_{5th})]$$

The resultant current density waveform is at a maximum near the proximal and distal ends 216a, 216b of the antenna 216. A relative maximum current density is also centered about the midpoint of the antenna 216. The current density is a minimum at the proximal and distal ends 216a, 216b of the antenna 216 and midway between the feedpoint and the maximum current density portions. In general, current density 342 is focused near the distal end of the antenna 216, near the proximal end of the antenna 216 and about the antenna 216 midpoint.

The resultant current density waveform 342 illustrated in FIG. 13 provide an example of a waveform that advantageously creates an ablation region through both microwave heating and conductive heating. The microwave heating creates a current density pattern 340 with focused energy delivery towards the proximal and distal ends 216a, 216b. The tissue portion at the feedpoint, midway between the maximum current density regions, receives energy from microwave heating and from energy conducted from the maximum current density regions proximal and distal the feedpoint.

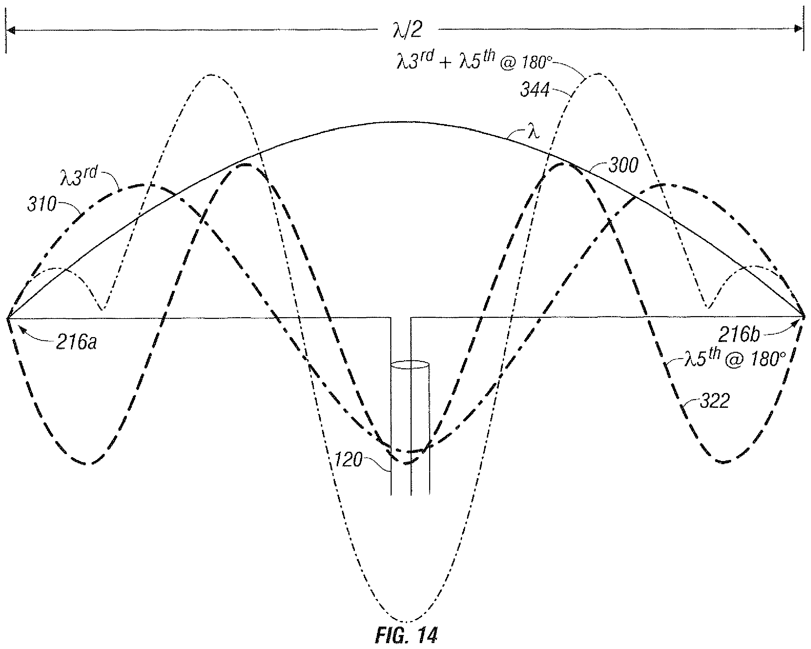

FIG. 14 illustrates current density plots of a microwave signal with a wavelength of $\lambda_{3rd}$ 310, supplied to Port A, a microwave waveform with a wavelength of $\lambda_{5th}$ 322 supplied to Port B and the resultant current density waveform 344. The waveforms at Ports A and B are phase-shifted by 180° on the $\lambda_{5th}$ scale and have a 1:1 intensity level. A microwave waveform with a wavelength of λ 300 is provided for reference. The magnitude of the resultant current density of the waveform at Port C is shown as 344 and is equal to:

$$\text{Abs} \left( \lambda_{3rd} + \lambda_{5th} \ @ \ 180° \right)$$

The current density is at a maximum at the midpoint of the antenna 216 with the current density for the $\lambda_{3th}$ 310 and $\lambda_{5th}$ waveforms 322 are a maximum. First pair of relative current density maximums are positioned about the midpoint of the proximal and distal radiating portions 216a, 216b with a second pair of smaller relative current density maximums near the proximal and distal ends of the antenna 216. The current density is a minimum between the maximum current density and the first pair of relative current density maximums and between the first and second pair of relative current density maximums. In general, the resultant current density waveform 344 extends well beyond the maximum current density of the $\lambda$ waveform 300. As such, the waveforms of $\lambda_{3rd}$ 310 + $\lambda_{5th}$ @ 180° 344 and the $\lambda$ waveform 300 may produce ablation regions of similar in size and shape.

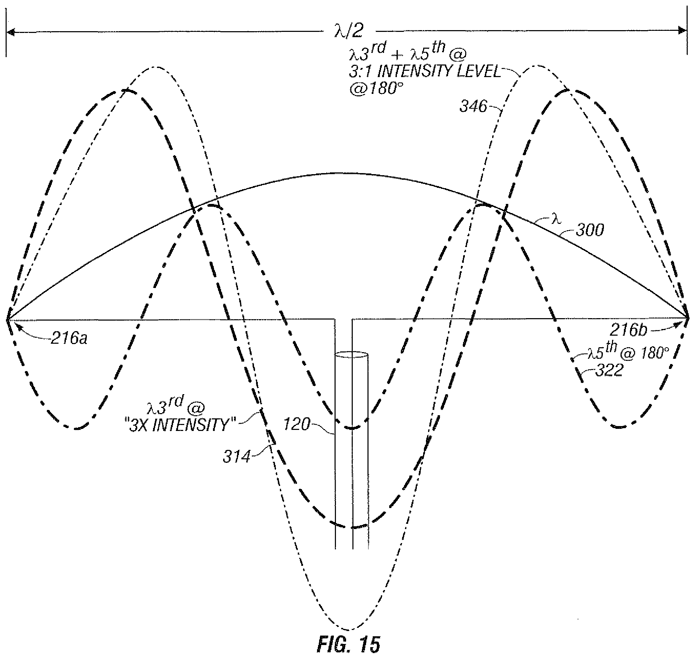

FIG. 15 illustrates current density plots of a microwave signal with a wavelength of $\lambda_{3rd}$ 314, supplied to Port A, a microwave signal with a wavelength of $\lambda_{5th}$ 322 supplied to Port B and the resultant current density waveform 346. The waveforms at Ports A and B are phase-shifted by 180° on the $\lambda_{5th}$ scale and have a 1:1 intensity level. A microwave waveform with a wavelength of $\lambda$ 300 is provided for reference. The magnitude of the resultant current density of the waveform at Port C is shown as 346 and is equal to:

$$\text{Abs} \left( (\lambda_{3rd} * 3) + \lambda_{5th} \ @ \ 180° \right)$$

The current density is at a maximum at the midpoint of the antenna with a pair of relative current density maximums positioned near the midpoint of the proximal and distal radiating portions 216a, 216b. The current density is a minimum between the maximum current density and each of the relative current density maximums. In general, the magnitude of current density 346 extends to, and beyond, the current density of the microwave waveform with a wavelength of $\lambda$ 300. As such, the waveforms of $\lambda_{3rd}$ *3 + $\lambda_{5th}$ @ 180° may produce ablation regions of similar in shape and size, if not larger size, than the waveform of $\lambda$ 300.

As illustrated in the FIGS. 10-15 and described hereinabove, combining microwave waveforms at various frequencies, phase relationships and intensities generate complex resultant current density waveforms. As such, the figures and waveforms provided hereinabove are for illustrative purposes and should not be construed as limiting.

In yet another embodiment of the present disclosure other tissue and/or energy properties may also be employed for determining or selecting the properties of the microwave signals, such as, for example, tissue temperature, power delivered to the surrounding medium and power reflected from the surrounding medium. In particular, the microwave generator may dynamically adjust one or more properties of energy delivery based on a measured value. Alternatively, the processor 151 in FIG. 5 may store a plurality of previously entered energy delivery parameter combinations, and the resulting current density patterns, and one or more of the energy delivery parameter combinations may be selected to produce an ablation region

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

**Claims**

1. A system (10) for delivering energy, comprising:

   a housing (112, 114) having an antenna (116) attached at the distal end thereof, the antenna configured to receive a microwave signal and radiate energy at at least two wavelengths, wherein the antenna is a di-pole antenna comprising proximal and distal radiating portions (216a, 216b); and
   a microwave generator (100) operable to provide the microwave signal to the antenna, wherein the microwave generator is operable to generate a combined microwave signal containing microwave energy having at least a first and a second wavelength; the microwave generator operable to deliver the combined microwave signal to the microwave antenna, wherein the first and second wavelengths are capable of creating resonance in the antenna, and

   wherein the microwave generator further includes:

   a first microwave signal generator (152a) operable to generate a first microwave signal at the first wavelength;
   a second microwave signal generator (152b) operable to generate a second microwave signal at the second wavelength;
   at least one phase shifter (154a, 154b) configured to shift the phase of one of the first and second microwave signals relative to each other;
   a signal mixer (158) that is operable to combine the first and second microwave signals to generate the combined microwave signal;
   a mixed signal amplifier circuit (159) comprising a signal measurement device for measuring forward and/or reflective power; and

a processor (151);

wherein the processor includes an algorithm configured to calculate or adjust the wavelength generated by the first and/or the second signal generator, wherein the mixed signal amplifier circuit is configured to provide forward and/or reflected power feedback to the processor and the processor is configured to calculate or adjust one or more properties of the generated signals, wherein the processor is configured to determine a first resonant frequency by varying the first wavelength generated by the first microwave signal generator and the processor is configured so that the second wavelength of the second microwave signal generator is determined by a similar algorithm or is calculated as a harmonic of the first wavelength.

2. The system according to Claim 1, wherein the at least one phase shifter is configured to shift the phase between the first and second microwave signals about 180°.

3. The system according to any one of the preceding claims, wherein the microwave generator further includes at least one amplifier (159) configured to amplify at least one of the first and the second microwave signals to a desired intermixing ratio.

4. The system according to Claim 3, wherein the intermixing ratio is between about 1:99 and 99:1.

5. The system according to any one of the preceding claims, wherein the first wavelength is related to a first frequency and the second wavelength is related to a harmonic of the first frequency.

6. The system according to Claim 5, wherein the harmonic is one of a third harmonic and a fifth harmonic.

7. The system according to Claim 5 or 6, wherein the first frequency is about 915 MHz.

8. The system according to any one of the preceding claims, further including a processor (151) configured to control a parameter of at least one of the first microwave signal, the second microwave signal, and the combined signal.

9. The system according to any one of the preceding claims, wherein the phase shifter is configured to change a phase relationship between the first and the second microwave signals during the delivery of energy in order to adjust the current density pattern or the shape of the ablation region.

10. The system of claim 9 so that hot spots, which are areas of concentrated energy at maximum current density position, of the antenna are continuously changing location.

11. The system of any one of the preceding claims, wherein the processor is configured to dynamically adjust the amount of the phase shift between the first and the second signals to maintain resonance with the antenna.

**Patentansprüche**

1. System (10) zur Lieferung von Energie, umfassend:

ein Gehäuse (112, 114) mit einer Antenne (116), die an einem distalen Ende davon befestigt ist, wobei die Antenne konfiguriert ist, um ein Mikrowellensignal zu empfangen und Energie mit zumindest zwei Wellenlängen abzustrahlen, wobei die Antenne eine Dipolantenne ist, die proximale und distale Strahlungsabschnitte (216a, 216b) umfasst; und

einen Mikrowellengenerator (100), der betreibbar ist, um das Mikrowellensignal an die Antenne bereitzustellen, wobei der Mikrowellengenerator betreibbar ist, um ein kombiniertes Mikrowellensignal zu erzeugen, das Mikrowellenenergie mit zumindest einer ersten und einer zweiten Wellenlänge aufweist; wobei der Mikrowellengenerator betreibbar ist, um das kombinierte Mikrowellensignal an die Mikrowellenantenne zu liefern, wobei die ersten und zweiten Wellenlängen in der Lage sind, Resonanz in der Antenne zu erzeugen, und wobei der Mikrowellengenerator weiter aufweist:

einen ersten Mikrowellensignalgenerator (152a), der betreibbar ist, um ein erstes Mikrowellensignal mit der ersten Wellenlänge zu erzeugen;

einen zweiten Mikrowellensignalgenerator (152b), der betreibbar ist, um ein zweites Mikrowellensignal mit der zweiten Wellenlänge zu erzeugen;

zumindest einen Phasenschieber (154a, 154b), der konfiguriert ist, um die Phase eines der ersten und zweiten Mikrowellensignale relativ zueinander zu verschieben;

einen Signalmischer, der betreibbar ist, um das erste und zweite Mikrowellensignal zu kombinieren, um das kombinierte Mikrowellensignal zu erzeugen;

eine Mischsignalverstärkerschaltung (159), die eine erste Signalmessvorrichtung zum Messen von Vorwärts- und/oder Reflexionsleistung umfasst; und

einen Prozessor (151);

wobei der Prozessor einen Algorithmus aufweist, der konfiguriert ist, um die von dem ersten und/oder dem zweiten Signalgenerator erzeugte Wellenlänge zu berechnen oder einzustellen, wobei die Mischsignalverstärkerschaltung konfiguriert ist, um Vorwärts- und/oder Reflexionsleistungs-Feedback an den Prozessor bereitzustellen, und der Prozessor konfiguriert ist, um eine oder mehrere Eigenschaften der erzeugten Signale zu berechnen oder einzustellen, wobei der Prozessor konfiguriert ist, um durch Variieren der von dem ersten Mikrowellensignalgenerator erzeugten ersten Wellenlänge eine erste Resonanzfrequenz zu bestimmen, und der Prozessor konfiguriert ist, so dass die zweite Wellenlänge des zweiten Mikrowellensignalgenerators durch einen ähnlichen Algorithmus bestimmt wird oder als Harmonische der ersten Wellenlänge berechnet wird.

2. System nach Anspruch 1, wobei der zumindest eine Phasenschieber konfiguriert ist, um die Phase zwischen dem ersten und dem zweiten Mikrowellensignal um etwa 180° zu verschieben.

3. System nach einem der vorstehenden Ansprüche, wobei der Mikrowellengenerator weiter zumindest einen Verstärker (159) aufweist, der konfiguriert ist, um zumindest eines der ersten und der zweiten Mikrowellensignale auf ein gewünschtes Mischungsverhältnis zu verstärken.

4. System nach Anspruch 3, wobei das Mischungsverhältnis zwischen etwa 1:99 und 99:1 liegt.

5. System nach einem der vorstehenden Ansprüche, wobei die erste Wellenlänge auf eine erste Frequenz bezogen ist und die zweite Wellenlänge auf eine Harmonische der ersten Frequenz bezogen ist.

6. System nach Anspruch 5, wobei die Harmonische eine von einer dritten Harmonischen und einer fünften Harmonischen ist.

7. System nach Anspruch 5 oder 6, wobei die erste Frequenz etwa 915 MHz ist.

8. System nach einem der vorstehenden Ansprüche, weiter aufweisend einen Prozessor (151), der konfiguriert ist, um einen Parameter von zumindest einem von dem ersten Mikrowellensignal, dem zweiten Mikrowellensignal und dem kombinierten Signal zu steuern.

9. System nach einem der vorstehenden Ansprüche, wobei der Phasenschieber konfiguriert ist, um eine Phasenbeziehung zwischen dem ersten und dem zweiten Mikrowellensignal während der Lieferung von Energie zu ändern, um das Stromdichtemuster oder die Form des Ablationsbereichs einzustellen.

10. System nach Anspruch 9, so dass Hotspots, die Bereiche mit konzentrierter Energie bei maximaler Stromdichteposition sind, der Antenne kontinuierlich ihren Ort ändern.

11. System nach einem der vorstehenden Ansprüche, wobei der Prozessor konfiguriert ist, um den Betrag der Phasenverschiebung zwischen dem ersten und dem zweiten Signal dynamisch einzustellen, um Resonanz mit der Antenne aufrechtzuerhalten.

**Revendications**

1. Système (10) pour délivrer de l'énergie, comprenant :

un boîtier (112, 114) ayant une antenne (116) attachée à son extrémité distale, cette antenne étant configurée pour recevoir un signal hyperfréquence et rayonner de l'énergie à au moins deux longueurs d'onde, dans lequel l'antenne est une antenne dipôle comprenant des parties rayonnantes proximale et distale (216a, 216b) ; et

un générateur hyperfréquence (100) opérationnel pour fournir le signal hyperfréquence à l'antenne,

dans lequel le générateur hyperfréquence est opérationnel pour générer un signal hyperfréquence combiné contenant de l'énergie hyperfréquence ayant au moins une première et une seconde longueur d'onde ; le générateur hyperfréquence étant opérationnel pour délivrer le signal hyperfréquence combiné à l'antenne hyperfréquence, les première et seconde longueurs d'onde pouvant générer une résonance dans l'antenne, et

dans lequel le générateur hyperfréquence comprend en outre :

un premier générateur de signal hyperfréquence (152a) opérationnel pour générer un premier signal hyperfréquence à la première longueur d'onde ;

un second générateur de signal hyperfréquence (152b) opérationnel pour générer un second signal hyperfréquence à la seconde longueur d'onde ;

au moins un déphaseur (154a, 154b) configuré pour décaler la phase de l'un des premier et second signaux hyperfréquences par rapport à l'autre ;

un mélangeur de signaux (158) qui est opérationnel pour combiner les premier et second signaux hyperfréquences afin de générer le signal hyperfréquence combiné ;

un circuit amplificateur de signal mixte (159) comprenant un dispositif de mesure de signal pour mesurer une puissance directe et/ou de réflexion ; et

un processeur (151) ;

dans lequel le processeur comprend un algorithme configuré pour calculer ou ajuster la longueur d'onde générée par le premier et/ou le second générateur de signal, dans lequel le circuit amplificateur de signal mixte est configuré pour fournir une rétroaction de puissance directe et/ou réfléchie au processeur, et le processeur est configuré pour calculer ou ajuster une ou plusieurs propriétés des signaux générés, dans lequel le processeur est configuré pour déterminer une première fréquence de résonance en modifiant la première longueur d'onde générée par le premier générateur de signal hyperfréquence et le processeur est configuré de sorte que la seconde longueur d'onde du second générateur de signal hyperfréquence est déterminée par un algorithme similaire ou est calculée en tant qu'harmonique de la première longueur d'onde.

2.  Système selon la revendication 1, dans lequel le au moins un déphaseur est configuré pour décaler la phase entre les premier et second signaux hyperfréquences d'environ 180°.

3.  Système selon l'une quelconque des revendications précédentes, dans lequel le générateur hyperfréquence comprend en outre au moins un amplificateur (159) configuré pour amplifier au moins l'un des premier et second signaux hyperfréquence à un rapport de mélange souhaité.

4.  Système selon la revendication 3, dans lequel le rapport de mélange est compris entre environ 1 :99 et 99 : 1.

5.  Système selon l'une quelconque des revendications précédentes, dans lequel la première longueur d'onde est liée à une première fréquence et la seconde longueur d'onde est liée à une harmonique de la première fréquence.

6.  Système selon la revendication 5, dans lequel l'harmonique est l'une d'une troisième harmonique et d'une cinquième harmonique.

7.  Système selon la revendication 5 ou 6, dans lequel la première fréquence est d'environ 915 MHz.

8.  Système selon l'une quelconque des revendications précédentes, comprenant en outre un processeur (151) configuré pour commander un paramètre d'au moins un parmi le premier signal hyperfréquence, le second signal hyperfréquence et le signal combiné.

9.  Système selon l'une quelconque des revendications précédentes, dans lequel le déphaseur est configuré pour changer une relation de phase entre les premier et second signaux hyperfréquences pendant la délivrance d'énergie afin d'ajuster le motif de densité de courant ou la forme de la région d'ablation.

10. Système selon la revendication 9, de sorte que des points chauds, qui sont des zones d'énergie concentrée à la position de densité de courant maximum, de l'antenne changent continuellement d'emplacement.

11. Système selon l'une quelconque des revendications précédentes, dans lequel le processeur est configuré pour ajuster dynamiquement la quantité du déphasage entre les premier et second signaux afin de maintenir une résonance avec l'antenne.

FIG. 1

**FIG. 2**

**FIG. 3**

*FIG. 4*

**FIG. 5**

EP 2 172 162 B1

FIG. 6A

FIG. 6B

*FIG. 7*

*FIG. 8A*

FIG. 8B

FIG. 9A

EP 2 172 162 B1

*FIG. 9B*

FIG. 10

FIG. 11

**FIG. 12**

EP 2 172 162 B1

FIG. 13

FIG. 14

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005205566 A1
- US 4448198 A1

- US 6878147 B, Prakash